(19) Europäisches Patentamt
European Patent Office
Office européen des brevets

(11) EP 1 005 342 B1

# (12) EUROPEAN PATENT SPECIFICATION

(45) Date of publication and mention of the grant of the patent:
**04.12.2002 Bulletin 2002/49**

(21) Application number: **98920285.8**

(22) Date of filing: **06.05.1998**

(51) Int Cl.[7]: **A61K 31/426**, A61P 31/04, A61P 33/00

(86) International application number:
**PCT/US98/09229**

(87) International publication number:
**WO 98/050035 (12.11.1998 Gazette 1998/45)**

(54) **PHARMACEUTICAL COMPOSITIONS OF TIZOXANIDE AND NITAZOXANIDE**

TIZOXANIDE UND NITAZOXANIDE ENTHALTENDE PHARMAZEUTISCHE ZUSAMMENSETZUNGEN

COMPOSITIONS PHARMACEUTIQUES DE TIZOXANIDE ET DE NITAZOXANIDE

(84) Designated Contracting States:
**AT BE CH DE DK ES FI FR GB GR IE IT LI LU MC NL PT SE**

(30) Priority: **07.05.1997 US 852447**
**03.07.1997 US 887810**
**03.07.1997 US 887809**

(43) Date of publication of application:
**07.06.2000 Bulletin 2000/23**

(60) Divisional application:
**02002887.4 / 1 222 921**

(73) Proprietor: **Romark Laboratories, L.C.**
**Tampa, FL 33607 (US)**

(72) Inventor: **ROSSIGNOL, Jean-François**
**St. Petersburg, FL 33710 (US)**

(74) Representative: **Winkler, Andreas, Dr.**
**FORRESTER & BOEHMERT**
**Pettenkoferstrasse 20-22**
**80336 München (DE)**

(56) References cited:
**US-A- 3 950 351**
**US-A- 4 315 018**
**US-A- 5 387 598**
**US-A- 5 578 621**

- **FEREGRINO-GOYOS ET AL: "clinical study of nitazoxamide in the therapy of cryptosporidium parvum in patients with aids" AIDS, vol. 10, no. 2, 1996, page s42 XP000943530**

Remarks:
The file contains technical information submitted after the application was filed and not included in this specification

EP 1 005 342 B1

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

Printed by Jouve, 75001 PARIS (FR)

## Description

### Field of the Invention

The present invention relates to a pharmaceutical composition containing as active agent at least one compound selected among the group consisting of formula (I)

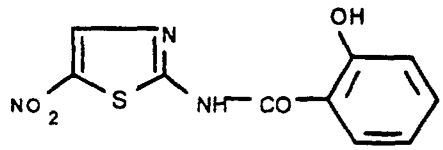

(I)

and formula (II)

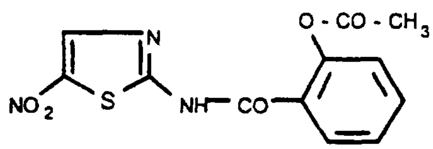

(II).

The active agent is preferably in the form of particles having a particle size smaller than 200 µm and a mean particle size of greater than 10 µm.

The invention also relates to pharmaceutical compositions stabilized with at least one pharmaceutically acceptable acid.

The pharmaceutical compositions are particularly useful for treatment of opportunistic infections in persons with compromised or suppressed immune systems, and in treatment of infections of trematodes.

### Description of the Related Art

There is an urgent need for the development of methods for treatment of a number of parasitic and bacterial infections in humans with compromised immune systems (AIDS, cancer patients, elderly, aging, organ transplant patients on immunosuppressive drugs). Another area of concern is trematode infections, particularly in tropical climates. There is thus a need for a pharmaceutical composition which can be tolerated by even immunocompromised humans, and which is storage stable even in tropical environments.

More specifically, *Toxoplasma gondii* is a protozoan and is among the most prevalent causes of latent infection of the central nervous system throughout the world. Many healthy people are infected with the parasite, but usually the immune system keeps the organism under control. *T. gondii* is the most common opportunistic pathogen of the brain in AIDS patients. At present, toxoplasmosis is becoming an increasing problem not only because of AIDS, but also because of wider use of immunosuppressive drugs (e.g., as administered to organ-transplant patients). Toxoplasmosis is usually treated with a combination of pyrimethamine and sulfadiazine. While the drugs are effective, they do not kill cysts of the parasite, so the treatment must be continued as a maintenance dose Toxicity often forces discontinuation of the drug, particularly in the immunosuppressed, and relapses result. The statistics are not good, with reported death rates of about 70 percent in immunodeficient patients and median survival of four months.

Cryptosporidiosis is caused by the microscopic protozoan parasite *Cryptosporidium parvum*. In persons with normal immune functions, the diarrhea caused by *C. parvum* may be intense and prolonged, but is self-limiting. In AIDS patients, cryptosporidial diarrhea is often life-threatening. It is estimated that 15-20 percent of AIDS patients suffer from this condition. Up to now, there has been no consistently effective or approved therapy for cryptosporidiosis.

The most frequently identified pathogen in AIDS patients is *Enterocytozoon bieneusi,* a microsporidian parasite, which was found in nearly one-quarter of the patients. It now appears that this tiny parasite may turn out to be the cause of a large proportion of the many unexplained cases of malabsorption, diarrhea and wasting seen in HIV-ill patients. There is no known effective treatment as yet.

Several other species of microsporidia infect HIV-positive patients, including *Encephalitozoon hellem* and *cuniculi,* and a new species designated *Septata intestinalis.* A recent report suggests that disseminated microsporidia

infections are increasing in significance.

Infection with the parasite *Isospora belli* is clinically indistinguishable from cryptosporidiosis. More common in tropical climates, *I. belli* has been reported in less than 1% of patients in the U.S., although its actual incidence is probably higher.

*Pneumocystis carinii* has generally been classified as a protozoan parasite; some studies indicate it may be a fungus, with which it shares certain genetic sequences. P. *carinii* usually infects the lungs (Pneumocystis Carinii Pneumonia (PCP)). Therapy is reported to be successful in about 40-60% of patients, with problems including drug toxicity particularly in immunocompromised patients. Among the many serious manifestations of human immunodeficiency virus (HIV) infection in children, PCP stands out because of its high incidence, unique age distribution, and frequent mortality. PCP is the most common serious opportunistic infection in children with HIV infection; the incidence of PCP among HIV-infected infants not receiving prophylaxis is estimated to be at least 12% in the first year of life. Many children die shortly alter PCP develops.

Mycobacterium Avium Complex (MAC) refers to infections by a family of very similar mycobacterial organisms, *Mycobacterium avium* and *M. intracellulare.* When MAC occurs in non-immunocompromised people, it is usually in the form of infection in the respiratory tract. In patients with AIDS, MAC is frequently disseminated (disseminated MAC or DMAC), and almost any organ system can be involved. In a recent study, MAC bacterial was found in 43% of patients who survived for 2 years after an AIDS diagnosis. No standard therapy has been established for disseminated MAC. Combinations of drugs are usually prescribed and, if successful, require that treatment be continued for life. A more effective treatment is urgently needed.

The HIV-infected are particularly susceptible to infection by *Mycobacterium tuberculosis,* and the course of the disease is accelerated. While extrapulmonary tuberculosis is unusual in non-HIV-infected patients, it frequently occurs in HIV-positive people. The CDC has released guidelines for the treatment of TB which address the growing prevalence of multi-drug resistant TB (MDR-TB). Mortality among AIDS patients with MDR-TB is very high (approximately 80%) and the disease progression is extremely rapid.

Accordingly, there is an urgent need for the development of a method of treatment of these infections so prevalent in, and threatening to, humans and animals.

There is also a need for a broad-acting drug for simplification of treatment of trematode infections. Presently, it is necessary to diagnose the specific trematode pathogen, and then to prescribe the drug therapy specific for that trematode. Many lesser developed countries are not equipped to diagnose a the specific trematode. Development of a broad acting drug would eliminate the requirement for diagnosis.

*Schistosoma mansoni,* the blood fluke, is the causative agent of Schistosomiasis, the second most important tropical parasitic disease of man (after Malaria), and the most important trematode infection of man. *Schistosoma haematobium* is another important species infecting man. Over 200 million individuals suffer from schistosomiasis world wide, including several hundred thousand people in the United States.

*Fasciola hepatica*, the common liver fluke, is primarily a disease of sheep, but humans are an accidental host. The parasite manages to survive in the presence of a vigorous host immune response. Bithionol has been suggested for treatment, but is not approved for use in the United States.

There is thus a need for a pharmaceutical composition which is storage stable even in tropical environments, and which is broad acting against trematodes.

In AIDS, 1996, 10 (2) the treatment of immunocompromised AIDS patients with daily oral doses of 1000 or 500 mg nitazoxanide, i.e. the compound of formula (II), is described.

## Summary of the Invention

It has now been observed in animal studies and in human clinical studies that the efficacy of a treatment, using the compounds of formula (I) and (II) is dependent upon the particle size of the active drug substance and the stability of the compounds.

The pharmaceutical compositions described are suitable for treating human and animal trematode infections caused by *Schistosoma* such as *Schistosoma mansoni, Schistosoma haematobium, Schistosoma mekongi, Schistosoma japonicum, Schistosoma intercalatum; Fasciola* such as *Fasciola hepatica* and *Fasciola gigantica, Fasciolopsis biski;* and *Dicrocoelium dendriticum, Heterophyes heterophyes,* and *Metagonimus yokogawa.*

The pharmaceutical compositions are also effective for treatment in the immuno-compromised of opportunistic infections of *Cryptosporidium parvum, Isospora belli, Enterocytzoon bieneusi, Encephalitozoon intestinalis, Mycobacterium tuberculosis, Mycobacterium avium intracellulare, Pneumocystis carinii*, and *Toxoplasma gondii.*

The pharmaceutical composition may be in a form suitable for oral administration, as a solid dosage form, a liquid suspension, or a paste.

## BRIEF DESCRIPTION OF THE DRAWINGS

For a fuller understanding of the nature and objects of the present invention reference should be made by the following detailed description taken in with the accompanying drawings in which:

Fig. 1 shows percent inhibition and host cell viability of nitazoxanide against *E. intestinalis.*
Fig. 2 shows percent inhibition and host cell viability of nitazoxanide against *V. corneae.*
Fig. 3 shows percent inhibition and host cell viability of albendazole against *E. intestinalis.*
Fig. 4 shows percent inhibition and host cell viability of albendazole against *V. corneae.*
Figs. 5 and 6 show a plot of OD values obtained for each *T. gondii* culture well, *vs* the concentration of the drug in the culture.
Fig. 7 is a chart based upon the assay for nitazoxanide effectiveness against mycobacteria growing in a liquid broth.
Fig. 8 shows the percentage of active particles having a size smaller than Ø µm.

## Detailed Description of the Invention

The present invention involves administration of a pharmaceutical composition comprising, as active agent, at least one compound selected from the group consisting of desacetyl-nitazoxanide of formula (I):

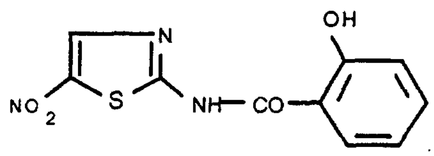

. (I)

and nitazoxanide of formula (II):

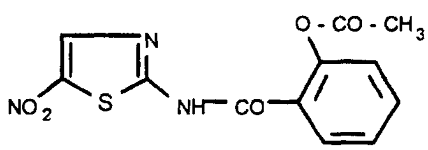

(II).

Nitazoxanide (NTZ), the compound of formula (II), is the generic name for 2-(acetolyloxy)-N-(5-nitro 2-thiazoly) benzamide, a compound first synthesized by Rossignol and Cavier in 1975. 2 mg of nitazoxanide can be dissolved in 1 ml DMSO. Nitazoxanide is easily absorbed orally.
Until now there has been no evidence that the compounds of formula (I) and/or (II) could be broadly effective against trematode infections, or that they would be sufficiently nontoxic to be tolerated by even immuno-compromised humans.
The preparation and certain uses of nitazoxanide is disclosed in US Patent 3,950,351, as well as in publications by the present inventor. Desacetyl-nitazoxanide, the compound of formula (I), is sometimes referred to as tizoxanide or d-NTZ, and is a metabolite of nitazoxanide.
In WO 95/28393, the present inventor disclosed a method for the manufacture of pure compound of formula (II), as well as the use of the composition containing a mixture of compounds of formula (I) and (II).
It has now been observed that solid particles of compound of formula (I), compound of formula (II), or mixtures thereof having a particle size between 170 and 520 µm (mean particle size = 352 µm) have very limited efficacy when administered orally to animals or humans. The efficacy of such particles is inferior to existing pharmaceutical products and therefore unacceptable for regulatory or commercial purposes.
It has also been observed in dogs that the oral administration of a single dose of 50 milligrams per kilogram of solid particles of compound of formula (I) and compound of formula (II) having a particle size smaller than 5 µm, caused severe adverse reactions in the animals.
It has now been discovered that in order to have an effective and safe treatment of infections caused by parasites, bacteria, fungi and viruses in humans and animals, the pharmaceutical composition, either a solid dosage

form or an aqueous suspension, must contain the effective dose of the active agent in the form of solid particles having a particle size smaller than 200 μm and containing compound of formula (I) and/or compound of formula (II), the mean particle size of the active solid particles being greater than 10 μm.

The presence of a high content of particles of active agent having a size larger than 200 μm with respect to the content of particles having a size between 5 and 200 μm significantly reduces the chemotherapeutic activity of the compounds. Preferably, the pharmaceutical compositions of the invention do not contain more than 5% by weight of active solid particles having a size larger than 200 μm. Most preferably, the pharmaceutical compositions of the invention contain substantially no active solid particles having a size larger than 200 μm.

The presence of a high content of particles of active agent having a size less than 5 μm with respect to the content of particles having a size between 5 and 200 μm can produce adverse effects in animals or in humans. In addition, it has been observed that particles having a size less than 5 μm are more rapidly absorbed from the gastro-intestinal tract into the bloodstream, and therefore are not as effective against parasites, bacteria, fungi and viruses which commonly live within the gastro-intestinal tract of animals and humans.

The skilled scientist could not predict that the particle size of compound of formula (I) and compound of formula (II) would have such a significant impact upon its antimicrobial activity in animals and in humans. For example, in studies conducted by the Inventor, anti-parasitic compounds such as albendazole, mebendazole, niclosamide, praziquantel and metronidazole have not demonstrated such a marked difference in anti-parasitic activity in animals or humans which was dependent upon their particle size. In addition, a skilled scientist could not predict that particle sizes of compound of formula (I) and compound of formula (II) would have such an adverse impact upon the ability of animals or humans to tolerate the administration of said active agent.

The compound(s) of formula (I) and (II) may be administered in either a solid dosage form or an aqueous suspension, and it is preferred that the pharmaceutical composition contain the effective dose of the active agent in the form of solid particles of formula (I) and/or (II) having a particle size smaller than 200 μm, the mean particle size of said active solid particles being greater than 10 μm as determined by a Coulters® Counter LS 100. This equipment uses laser light at 750 nm to size particles from 0.4 to 900 μm in diameter by light diffraction. The samples are measured in water with a small amount of Triton X-100 in order to increase the wettability and deflocculate the powder.

Advantageously, the mean particle size of said active solid particles is between 10 and 100 μm, preferably between 20 and 50 μm. Examples of preferred compositions are:

- a composition for which less than 10% by weight of said active solid particles has a particle size larger than 100 μm;
- a composition for which at least 50% by weight of said active solid particles has a particle size smaller than 50 μm.

Advantageously, the mean particle size of said active solid particles is between 10 and 100 μm, preferably between 20 and 50 μm. In accordance with a preferred embodiment of the composition, less than 10% of said active solid particles has a particle size smaller than 5 μm.

The active agent or agents used in the solid dosage form or suspension is advantageously a mixture of solid particles of compounds of formula (I) and of formula (II) with a particle size smaller than 200 μm, the weight content of compound of formula (I) with respect to the weight of compounds of formula (I) and of formula (II) of said mixture being comprised between 0.5 and 20%, preferably between 0.5 and 10%.

The invention also relates to pharmaceutical compositions described above which contain advantageously at least one pharmaceutically acceptable acid. Examples of such acids are: citric acid, glutamic acid, succinic acid, ethanesulfonic acid, acetic acid, tartaric acid, ascorbic acid, methanesulfonic acid, fumaric acid, adipic acid, malic acid and mixtures thereof. Citric acid is very appropriate. The presence of said acid improves the stability of the active agent or agents.

The ratio of the weight of pharmaceutically acceptable acid/the weight of said active solid particles is advantageously between 0.01 and 0.5, preferably between 0.03 and 0.2. Advantageously, the amount of acid is sufficient for adjusting the pH of the suspension between 2 and 6, preferably between 3 and 5, most preferably between 3.5 and 4.5.

Techniques for preparation of, and preferred examples of, solid and liquid dosage forms of the pharmaceutical composition are disclosed in WO/95/28393. The compositions contain advantageously a wetting agent and possibly a starch derivative such as those disclosed in US Patent 5,578,621, the content of which is incorporated herein by reference for disclosing possible wetting agents and starch derivatives. The wetting agent as described in US 5,578,621 serves as a dispersing agent.

Such pharmaceutical compositions, either as solid or liquid dosage forms or as pastes or ointments, can optionally contain additional active agents such as antibiotics, antiviral agents or proton pump inhibitors. While it is not advantageous, it is also possible that such pharmaceutical formulations contain active solid particles of compound of formula (I) and/or compound of formula (II) which are larger than 200 μm.

The compositions can contain excipients known as such for the purpose of preparing forms suitable for oral administration.

Advantageously, in order to have excellent efficacy against a broad spectrum of parasites, bacteria, fungi and viruses, the distribution factor of said active solid particles is between 0.8 and 2, preferably between 1.1 and 1.9, most preferably greater than 1.5, said distribution factor being calculated by the following formula:

$$F_{90\%} = (Ø_{90\%} - Ø_{10\%})/((Ø_{90\%} + Ø_{10\%}) /2)$$

in which

- $F_{90\%}$ is the distribution factor at 90%;
- $Ø_{90\%}$ is the maximum particle size of the fraction of particles corresponding to 90% of said active solid particles, and
- $Ø_{10\%}$ is the maximum particle size of the fraction of particles corresponding to 10% of said active solid particles.

According to a specific embodiment of the invention, particles of a compound of formula (I) and/or (II) are prepared by the methods described hereabove and are then milled so that less than 10% of said active particles are larger than 100 µm, less than 50% of said particles are larger than 50 µm and less than 10% of said active particles are smaller than 5 µm in size, the mean particle size being between 20 and 50 µm. Said active particles are then granulated using a mixture containing active solid particles and at least one granulating agent. Examples of granulating agent are: polyvinylpyrrolidone, water, alcohol, sucrose, hydroxyl cellulose and mixtures thereof. Advantageously, at least one pharmaceutically acceptable acid is added during the granulation process.

The invention relates to solid dosage forms containing a composition of the invention such as tablets, dispersible tablets, coated tablets, matrixes, etc. The dosage form of the invention contains, for example:

- solid active particles with a particle size smaller than 200 µm, less than 10% of said particles having a size larger than 100 µm, less than 50% of said particles having a size larger than 50 µm and less than 10% of said particles having a size less than 5 µm, the mean particle size being between 20 and 50 µm.
- at least one granulating agent;
- at least one wetting agent;
- at least one starch derivative, and
- at least one pharmaceutically acceptable acid which is preferably added during the granulation process.

Liquid dosage forms such as aqueous suspensions of the invention contain, for example:

- as active agent, solid particles containing a compound of formula (I) and/or a compound of formula (II) having a particle size smaller than 200 µm, less than 10% of said particles having a size larger than 100 µm, less than 50% of said particles having a size larger than 50 µm and less than 10% of said particles having a size less than 5 µm, and
- at least one granulating agent;
- at least one wetting agent;
- at least one pharmaceutically acceptable acid, the pH of the suspension being between 2 and 6, preferably between 3 and 5, most preferably between 3.5 and 4.5;
- at least one thickener, for example a Xanthan gum, a guar gum, crystalline cellulose, carruba gum, carboxymethylcellulose or a mixture thereof.

Paste or ointment forms of the invention suitable for oral administration contain, for example:

- as active agent, solid particles containing a compound of formula (I) and/or a compound of formula (II) having a particle size smaller than 200 µm, less than 10% of said particles having a size larger than 100 µm, less than 50% of said particles having a size larger than 50 µm and less than 10% of said particles having a size less than 5 µm, and
- at least one wetting agent;
- at least one pharmaceutically acceptable acid, the pH of the suspension being between 2 and 6, preferably between 3 and 5, most preferably between 3.5 and 4.5;
- at least one thickener, for example a Xanthan gum, a guar gum, crystalline cellulose, carruba gum, carboxymethylcellulose or a mixture thereof.

Paste or ointment forms for topical or intravaginal application contain, for example:

- as active agent, solid particles containing a compound of formula (I) and/or a compound of formula (II) having a particle size smaller than 200 µm, less than 10% of said particles having a size larger than 100 µm, less than 50%

of said particles having a size larger than 50 µm and less than 10% of said particles having a size less than 5 µm, and

- at least one wetting agent;
- at least one pharmaceutically acceptable acid, the pH of the suspension being between 2 and 6, preferably between 3 and 5, most preferably between 3.5 and 4.5;
- cetylic alcohol and/or glyceride derivatives and/or propyleneglycol;
- at least one thickener, for example a Xanthan gum, a guar gum, crystalline cellulose, carruba gum, carboxymethylcellulose or a mixture thereof.

**Description of Preparation of Pharmaceutical Compositions**

Dry pure compound of formula (I) and dry pure compound of formula (II) were submitted to grinding and sized by means of a mesh screen.

After grinding, the particles of compound of formula (I), of formula (II) and mixtures thereof had the particle size distribution as given in Fig. 8. Fig. 8 shows the percentage of particles having a size smaller than Ø µm.

From said figure, it appears that:

- less than 10% by weight of the particles had a particle size smaller than approximately 5 µm;
- less than 10% by weight of the particles had a particle size larger than approximately 70 µm;
- the mean particle size is approximately 40 µm;
- the distribution factor of the particles is about 1.73, said distribution factor being calculated by the following formula:

$$F_{90\%} = (Ø_{90\%} - Ø_{10\%})/((Ø_{90\%} + Ø_{10\%})/2)$$

in which

- $F_{90\%}$ is the distribution factor at 90%;
- $Ø_{90\%}$ is the maximum particle size of the fraction of particles corresponding to 90% of said active solid particles, and
- $Ø_{10\%}$ is the maximum particle size of the fraction of particles corresponding to 10% of said active solid particles.

Specific examples of such compositions are disclosed in the following Tables.

Table 1.

| **Example of composition of dispersible tablets for oral administration containing compound of formula (II) and compound of formula (I) as active agents.** | |
|---|---|
| Nitazoxanide (99%) + desacetyl-nitazoxanide (1%) | 200 mg |
| Microcrystalline cellulose Avicel pH 102 sold by FMC-USA | 116 mg |
| Crospovidone | 25 mg |
| Magnesium stearate | 3 mg |
| Colloidal silicon dioxide | 5 mg |
| Citric acid | 10 mg |
| Strawberry flavor No. 877720 sold by Robertet | 10 mg |
| Sodium saccharinate | 2 mg |

Table 2.

| **Example of composition of coated tablets for oral administration containing compound of formula (II) and compound of formula (I) as active agents.** | |
|---|---|
| Nitazoxanide | 500 mg |
| Maize starch | 60 mg |
| Pregelatinized Maize starch | 70 mg |
| Hydroxypropyl methylcellulose | 5 mg |
| Sucrose | 20 mg |
| Sodium starch glycollate | 30 mg |
| Citric acid | 25 mg |

Table 2. (continued)

| Example of composition of coated tablets for oral administration containing compound of formula (II) and compound of formula (I) as active agents. | |
|---|---|
| Talc | 8 mg |
| Magnesium stearate | 7 mg |

Coatings:

Hot sugar solution or a film coating being sprayed on the tablets or granules containing 500 mg active agent

Table 3.

| Example of an aqueous suspension for oral administration containing compound of formula (II) and compound of formula (I) as active agents. The pH of the suspension was about 4.1. | |
|---|---|
| Nitazoxanide (98%) + desacetyl-Nitazoxanide (2%) | 2 g |
| Distilled water | 100 ml |
| Sodium benzoate | 0.2 g |
| Saccharose | 30.5 g |
| Xanthan gum | 0.2 g |
| Microcrystalline cellulose and carboxymethylcellulose sodium Avicel RC-591 sold by FMC - USA | 0.8 g |
| Citric acid | 0.2 g |
| Dihydrated sodium citrate | 50 mg |
| Strawberry flavor No. 877720 sold by Robertet | 125 mg |
| Red dye No. 33 D and C | 1 mg |

Table 4.

| Example of a paste for oral administration containing compound of formula (II) and compound of formula (I) as active agents. | |
|---|---|
| Nitazoxanide (98%) + desacetyl-Nitazoxanide (2%) | 500 mg |
| Mineral oil | 10 g |
| Brown sugar | 1 g |
| Microcrystalline cellulose and carboxymethylcellulose sodium Avicel RC-591 sold by FMC | 0.8 g |
| Citric acid | 0.2 g |

Table 5.

| Example of a paste or ointment formulation for intravaginal or topical application, said paste or ointment containing compound of formula (II) and compound of formula (I) as active agents. | |
|---|---|
| Nitazoxanide (98%) + desacetyl-Nitazoxanide (2%) | 8 g |
| Cremaphor A6 | 2 g |
| Cremaphor A25 | 1.5 g |
| Mineral oil | 7 g |
| Luvitol EHO | 7 g |
| Glycerol monoester | 4 g |
| Cetylic alcohol | 3 g |
| Simeticone | 0.5 g |
| Germaben II | 1 g |
| Propyleneglycol | 3.5 g |
| Distilled water | 62.5 g |

The pharmaceutical compositions of the invention are compositions having a broad spectrum of action on

parasites, bacteria, fungi and viruses, especially when administered orally.

The efficacy and the safety of the pharmaceutical compositions disclosed hereabove were excellent in animals and in humans. Specifically, in human clinical studies, it has been observed that the efficacy of the pharmaceutical compositions described hereabove are significantly more effective in treating parasitic infections than the same formulations using active compound having particle sizes between 170 and 520 μm (mean particle size = 352 μm), even when the larger sized particles were administered to patients at doses up to three times higher and for longer periods of time. Examples of cure rates obtained are shown below in Table 6.

Table 6.

| **Comparison of results of human clinical studies using compounds of formula (I) and formula (II) having particle sizes ranging from 170 μm to 520 μm (mean = 352 μm) with results obtained using formula (I) and formula (II) having particle sizes ranging from 5 μm to 200 μm (mean = 34 μm)**. | | |
|---|---|---|
| Compound of formula (I) (98%) + Compound of formula (II) (2%) | | |
| | ***Particle sizes 170 to 520 μm Dose = 15 to 50 mg/kg/day for 3 to 7 days*** **No. Cured/Total = % Cure Rate** | ***Particle sizes 5 to 200 μm Dose = 15 mg/kg/day for 3 days*** **No. Cured/Total = % Cure Rate** |
| **Parasite** | | |
| Blastocystis hominis | 12/27 = 44% | 10/10 = 100% |
| Entamoeba histolytica | 29/47 = 62% | 106/133 = 80% |
| Giardia lamblia | 11/37 = 30% | 50/73 = 68% |
| Ascaris lumbricoides | 3/69 = 4% | 144/179 = 80% |
| Trichuris trichiura | 7/48 = 15% | 58/79 = 73% |

For each of the parasites listed in Table 6, the proportional cure rates were significantly better for the patients treated with active particles between 5 and 200 μm than for those treated with active particles ranging from 170 μm to 520 μm in size, with a statistical significance in each case being $p < 0.02$ (using a standard $X^2$ test). This was the case even though the doses of the larger particle size active agent were usually higher and the duration of treatment was often longer than that administered to the patients receiving pharmaceutical compositions of active agent having particle sizes smaller than 200 μm. There were no serious adverse effects reported for either group of patients.

Results similar to those described above for human studies have also been observed in animal testing.

In addition, the adverse reactions observed in dogs after oral administration of a single dose of 50 milligrams per kilogram of compound of formula (I) and compound of formula (II) have not been observed in extensive studies in animals using compound of formula (I) and compound of formula (II) having a particle size between 5 and 200 μm (mean > 10 μm), even when the same dose or a higher dose of the compounds were administered daily for 90 days or longer.

Moreover, said compositions were stable (even when subjected to temperatures of 40° C and 65% relative humidity for six months or, in the case of liquid suspensions, when suspended in water under these conditions for 3 months) thereby assuring that the active ingredients do not degrade and that the compositions maintain their efficacy for a period of time after their preparation which is suitable for medicinal and commercial purposes.

In the following, efficacy of the pharmaceutical compositions will be demonstrated.

## EXAMPLE I

*CRYPTOSPORIDIUM PARVUM*

In a preliminary clinical trial, 30 AIDS patients with chronic cryptosporidial diarrhea were treated with oral nitazoxanide from 500 to 2000 mg daily. If the diarrhea continued, the patients received an additional four weeks of nitazoxanide, up to 2000 mg a day.

Twenty-eight people completed two or more weeks of therapy and 16 of those were evaluable for a therapeutic response by the eighth week of treatment. In this latter group, 12 persons had a 50 percent or greater reduction in

daily bowel movement frequency and 10 individuals had a marked reduction or eradication of the parasite in the stool, with the organism becoming undetectable in four people. Six patients met both clinical and parasitological response criteria for benefit.

Patients who received higher daily doses of drug for longer periods of time were more likely to have a positive response.

An open-label study of nitazoxanide for AIDS-related cryptosporidial diarrhea documented decreased bowel movements among persons taking 500, 1000, 1500, or 2000 mg of the drug daily. Trial participants had a mean CD4+ count of 42 cells/$mm^3$ (range 0-303 cells/$mm^3$), a mean 6.7 bowel movements daily for an average 15 months, *Cryptosporidium parvum* oocysts in stool, and no other apparent enteric pathogens. Almost all participants had failed therapy with azithromycin or paromomycin.

After 23 weeks, 9 of 13 had a complete clinical response (one to three predominantly formed bowel movements daily), and 4 of 13 had a partial clinical response (at least a 50 percent decrease in daily bowel movements or a change in stool consistency so that at least 75 percent were formed) . By the end of the study, 8 of 11 had completely eradicated the parasite and the other three had substantial reductions in oocyst levels. There was a trend toward better response with doses of 1000 mg daily or higher and with longer therapy. Two trial participants had urticarial skin rashes; more than 90 percent adhered to the study regimen for more than four weeks.

## EXAMPLE II

*CRYPTOSPORIDIUM PARVUM*

*In vitro* Dose Information:

Nitazoxanide was dissolved in sterile dimethylsulfoxide (DMSO) and tested against intact *C. parvum* oocysts infected cell monolayers at concentrations of 100 µg/ml, 10 µg/ml/l µg/ml and 0.1 µg/ml. A second trial was performed which tested nitazoxanide at the additional concentrations of 20, 2, 0.2 and 0.02 µg/ml. These concentrations were achieved by serial dilutions with complete DMEM medium to yield a final DMSO concentration of 0.5%. The medium control also contained 0.5% DMSO.

The experiment used a cell culture of MDBKF5D2 Cells grown in 7 mm chambers, and as *Cryptosporidium parvum*: GCH1 oocysts, 5 X $10^4$ per well, and was conducted to compare paromomycin (positive control) against nitazoxanide (experimental drug). Materials included Immune Anti-*Cryptosporidium parvum* Sporozoite Rabbit Serum (0.1%) and Fluorescein-Conjugated Goat Anti-Rabbit Antibody (1%).

Toxicity Testing Assay:

200µl of medium containing nitazoxanide at concentrations of 100, 10, 1 and 0.1 µg/ml and the proper controls were introduced into two wells of a 96 well plate containing confluent MDBKF5D2 cell monolayers and two wells without monolayers. The drug was incubated on the monolayers at 37°C and 8% $CO_2$. At 24 hours (trial 1) and 48 hours (trial 2), MTS (Owen's solution) and PMS were added to each well at concentrations of 333 µg/ml and 25 µM respectively. The plate was returned to the incubator in the dark to develop for two hours. At two hours, 100 µl of each supernatant was transferred to a new microtiter plate and read in the ELISA reader at 490nm. Results were recorded and analyzed. Percent toxicity was calculated by subtracting the mean optical density (OD) of the drug supernatants from the mean optical density (OD) of the medium control supernatants (no drug), dividing by the OD of the medium control and multiplying by 100.

$$\frac{\text{OD medium - OD drug}}{\text{OD medium}} \times 100$$

Intact *C. parvum* Oocyst Assay:

5 x 104 *C. parvum* oocysts per well were incubated in nitazoxanide (100, 20, 10, 2, 1, 0.2, 0.1 and 0.02 µg/ml) at 37°C (8% CO2) on confluent MDBKF5D2 cell monolayers. The level of infection in each well was determined and analyzed by an immunofluorescence assay at 24 to 48 hours. Percent Inhibition was calculated by subtracting the mean parasite count/10 fields in the drug test wells from the mean parasite count/10 fields in the medium control (no drug), dividing by the medium control count, and then multiplying by 100.

$$\frac{\text{Medium control count - Drug test count}}{\text{Medium control count}} \times 100$$

Results:

| Trial 1: 24 hrs. | | | | |
|---|---|---|---|---|
| **Compound** | **Conc.** | **Mean (+ SD)*** | **Percent Toxicity** | **Percent Inhibition** |
| **Infected Media** | 0 | 983.5(±128.2) | 0 | 0 |
| **Paromomycin** | 2 mg/ml | 482(±47.1) | 23.8 | 51 |
| **NTZ** | 100µg/ml | Lost | 88.1 | NA** |
| | 10µg/ml | 55.5(±13.5) | 65.1 | 94.4 |
| | 1µg/ml | 224.5(±28.5) | 8.3 | 77.2 |
| | 0.1µg/ml | 474.5(±29.5) | 19.3 | 51.8 |

* Parasite Count/10 Fields

**Not available due to toxicity

| Trial 2: 48 hrs. | | | | |
|---|---|---|---|---|
| **Compound** | **Conc.** | **Mean (+ SD)*** | **Percent Toxicity** | **Percent Inhibition** |
| **Infected Media** | 0 | 2231.25(+90.03) | 0 | 0 |
| **Paromomycin** | 2 mg/ml | 580 (+33.42) | 40.8 | 74.01 |
| **NTZ** | 20µg/ml | 68.75(+13.77) | 92.87 | 96.92 |
| | 2µg/ml | 113.75(+21.36) | 24.93 | 94.90 |
| | 0.2µg/ml | 1020(+158.48) | 16.56 | 54.29 |
| | 0.02µg/ml | 1041(+191.46) | 21.23 | 53.33 |

* Parasite Count/10 Fields

Impact of nitazoxanide on the intact *C. parvum* oocysts:

In trial 1, nitazoxanide at concentrations of 10, 1 and 0.1 resulted in parasite inhibition levels of 94.4, 77.2 and 51.8%, respectively, and cell toxicity levels of 65.1, 8.3 and 19.3% respectively. Although nearly complete inhibition of parasite infection occurred in 10 µg/ml, a high toxicity rating was evident. At 1 µg/ml of nitazoxanide, parasite inhibition and cellular toxicity compared favorably to paromomycin at a concentration of 2 mg/ml (77.2% parasite inhibition and 8.3% toxicity for nitazoxanide at 1 µg/ml compared to 51% parasite inhibition and 23.8% cell toxicity for paromomycin at 2 mg/ml).

In trial 2, the drug was modified to obtain better dose distribution with minimum toxicity. Consequently, the cultures remained viable for 48 hours instead of 24 hours as in trial 1. Incubation for 48 hours clearly resulted in higher relative cell toxicity as evident from examination of paromomycin in both trials. The 20 µg/ml concentration of nitazoxanide was still too toxic at 48 hours incubation although the cell monolayer appeared still intact. It is possible that high toxicity which must affect cell function also impacts parasite infection/development. At 2 µg/ml of nitazoxanide, there was a considerable inhibition of the parasite infection with relatively low cellular toxicity. Further dilutions also resulted in significant inhibition and low toxicity. At a drug concentration of 2 µg/ml, moderate cell toxicity and inhibitory activity of 94.90% indicates that nitazoxanide at 2 µg/ml is superior to paromomycin for *in vitro C. parvum* infection at 2 mg/ml (e.g. 1000 times higher concentration).

**EXAMPLE III**

*CRYPTOSPORIDIUM PARVUM*

*In vitro* Dose and Storage Information:

Stocks of nitazoxanide and desacetyl-nitazoxanide (NTZ and NTZdes) were tested against intact *C. parvum* oocysts and excysted sporozoite infected cell monolayers at concentrations 10, 1, 0.1 and 0.01 µg/ml. Each compound was dissolved in 100% dimethyl sulfoxide (DMSO) and diluted to the desired concentrations with sterile DMEM. Each concentration of nitazoxanide and the media controls contained 0.025% DMSO as a constant.

The experiment used a cell culture of MDBKF5D2 Cells grown in 7 mm chambers, and as *Cryptosporidium parvum*: GCH1 oocysts, 5 X $10^4$ per well, and was conducted to compare paromomycin (positive control) against nitazoxanide (experimental drug). Materials included Immune *Anti-Cryptosporidium parvum* Sporozoite Rabbit Serum (0.1%) and Fluorescein-Conjugated Goat Anti-Rabbit Antibody (1%).

Toxicity Testing Assay:

200µl of medium containing nitazoxanide solution at the pre-mentioned concentrations and the proper controls were introduced into two wells of a 96 well plate containing confluent MDBKFD2 cell monolayers and two wells without monolayers. The drug was incubated on the monolayers at 37°C and 8% $CO_2$. At 48 hours, MTS (Owen's solution) and PMS were added to each well at concentrations of 333 µg/ml and 25 µM respectively. The plate was returned to the incubator in the dark to develop for 2 hours. At 2 hours, 100 µl of each supernatant was transferred to a new microtiter plate and read in the ELISA reader at 490 nm. Results were recorded and analyzed. Percent toxicity was calculated by subtracting the mean optical density (OD) of the drug supernatants from the mean OD of the medium control supernatants (no drug), dividing by the OD of the medium control and multiplying by 100.

$$\frac{\text{OD medium - OD drug}}{\text{OD medium}} \times 100$$

Cytotoxicity scores were assigned as follows: 0.5% toxicity = $0^-$, 6-25% toxicity = 1, 26-50% toxicity = 2, 51-75% toxicity = 3 and 76-100% toxicity =- 4. As a standard, cytotoxicity scores of 0 or 1 are to be considered acceptable levels of toxicity. Toxicity scores of 2, 3 or 4 are considered a high level or toxicity to the cell monolayer.

Intact C. *parvum* oocyst Assay:

5 x 104 C. *parvum* oocysts per well are incubated in the pre-mentioned concentrations of nitazoxanide at 37°C (8% $CO_2$) on confluent MDBKF5D2 cell monolayers. The level of infection in each well was determined and computer analyzed by an immunofluorescence assay at 48 hours. Percent Inhibition was calculated by subtracting the mean parasite count/field in the drug test wells from the mean parasite count/field in medium control (no drug), dividing by the medium control count and multiplying by 100.

$$\frac{\text{Medium control count - Drug test count}}{\text{Medium control count}} \times 100$$

Results:

| *C. parvum* Oocysts Assay (48 hr.) | | | | | | | | |
|---|---|---|---|---|---|---|---|---|
| **Drugs** | **Conc** | **Parasite** | **±SD** | **Tox/OD** | **±SD** | **%Inhib** | **%Tox** | **Score** |
| **Aqueous Media** | 0 | 681.58 | ±271.02 | 2.024 | ±0.18 | 0 | 0 | 0 |
| **Paromomycin** | 2000 | 115.75 | ±44.65 | 1.219 | ±.009 | 83.02 | 39.79 | 2 |

(continued)

| C. parvum Oocysts Assay (48 hr.) | | | | | | | | |
|---|---|---|---|---|---|---|---|---|
| **Drugs** | **Conc** | **Parasite** | **±SD** | **Tox/OD** | **±SD** | **%Inhib** | **%Tox** | **Score** |
| **0.025% DMSO Media** | 0 | 628.50 | ±171.94 | 1.799 | ±1.45 | 0 | 0 | 0 |
| **NTZ** | 10 | 11.75 | ±7.33 | .413 | ±0.13 | 98.13 | 77.07 | 4 |
| | 1 | 39.67 | ±13.13 | 1.618 | ±.326 | 93.69 | 10.09 | 1 |
| | 0.1 | 643.42 | ±229.73 | 1.878 | ±.154 | ≤0 | ≤0 | 0 |
| | 0.01 | 714.33 | ±194.79 | 1.617 | ±.072 | ≤0 | 10.12 | 1 |
| **New NTZdes** | 10 | 13.75 | ±6.66 | .337 | ±.005 | 97.81 | 81.27 | 4 |
| | 1 | 39.92 | ±13.49 | 1.710 | ±.033 | 93.65 | 4.97 | 0 |
| | 0.1 | 649.86 | ±152.19 | 1.506 | ±.119 | ≤0 | 16.29 | 1 |
| | 0.01 | 749.33 | ±139.49 | 1.721 | ±.144 | ≤0 | 4.36 | 0 |
| Conc. - μg/ml; Parasite - Mean parasite count/field (12 fields analyzed); %Inhib - Percent inhibitation of parasite infection; % Tox - Percent toxicity to cells by the drug. | | | | | | | | |

It can be seen from the above that the Inhibitory activity of NTZdes was the same as NTZ of Example II. Both nitazoxanide and desacetyl nitazoxanide were equally effective in vitro against *Cryptosporidium parvum* when tested in parallel with 98 and 94% inhibitions obtained with 10 and 1 μg/ml for each compound respectively. For nitazoxanide 1 μg/ml was the lowest concentration giving more than 90% of inhibition while 50% inhibition could be obtained with lower concentrations of nitazoxanide such as 0.2, 0.1 and 0.02 μg/ml. In the same experimental condition paromomycin used as positive control was 2,000 times less effective with inhibitory concentrations ranging from 51 to 83% at a concentration of 2,000 μg/ml.

## EXAMPLE IV

*E. INTESTINALIS AND V. CORNEA*

2RK-13 cells (rabbit kidney cell line) were added to 24-well culture plates at a concentration of 2.6 x $10^5$ cells per well (1.0 ml medium; RPMI 1640 with 2 mM L-glutamine and 5% heat-inactivated fetal bovine serum). Dishes were incubated at 37°C in a $CO_2$ incubator overnight at which time the wells were confluent (with one doubling, one would estimate 5 x $10^5$ cells per well).

Septata *intestinalis* (tissue culture-derived) organisms were added to the host cells at a 3:1 ratio compared with the estimated host cells or at 15. x $10^6$ organisms per well. This ratio resulted in approximately 50% of the host cells becoming infected.

Drugs were dissolved in DMSO, water or methanol (depending on solubility) to generate stocks of 1.0 mg/ml. Stocks were stored at -70°C. Dilutions used in experiments are made in complete tissue culture medium. All dilutions are tested in triplicate well.

Medium is replaced every three-to-four days (containing freshly-diluted drugs).

On day six (after adding parasites and drugs), the cells are examined for toxicity. Control cells given drugs but no parasites are examined for confluency, morphology of cells, and presence of dead or floating cells. Cells incubated with parasites only are examined to confirm that parasites are infectious (i.e. presence of parasitophorous vacuoles). Cells incubates the parasites and drugs are evaluated for host cell toxicity and relative numbers of parasitophorous vacuoles (i.e. high, medium, or low).

On day ten, 100 μl of 10% SDS (0.5% final concentration) was added to the culture wells to disrupt host cell membranes and cause release of the microsporidia. The total number of parasites present in each well was determined by counting an aliquot on a hemacytometer. Results are expressed as percent inhibition (relative to infected cells given no drug).

The results are shown in Figs. 1-4.

**EXAMPLE V**

*TOXOPLASMA GONDII*

Nitazoxanide and desacetyl nitazoxanide were tested against parasites, and more specifically, RH strain of *Toxoplasma gondii,* maintained by serial passages in mice. Cell cultures of MRC5 fibroblasts (Bio-Merieux, France) cultured in 96-well microplates were inoculated with *T. gondii.* 200 fresly harvested tachyzoites were added into each culture well, except in 8 control wells (negative controls). After 4 hours of incubation, drug dilutions were added into the cultures.

Nitazoxanide (NTZ) and desacetyl nitazoxanide (dNTZ) were tested at concentrations ranging between $8.10^{-4}$ and 40 mg/L. Drugs were initially dissolved in DMSO, at a concentration of 2 mg/mL, then serial dilutions were prepared in the culture medium. No precipitate was observed.

Drug dilutions were added into the cultures (8 wells for each dilution) then culture plates were incubated for 72 hours. Cultures were then fixed with cold methanol. Assessment of growth of *T. gondii* was performed buy ELISA using a peroxydase labeled rabbit anti *T. gondii* antibody. Optical density values were recorded for each well.

Resuts are presented by plotting the OD values obtained for each culture well, *vs* the concentration of the drug in the culture. Statistical analysis consisted in regression analysis with 95% confidence interval and determination of dose-response curves, from the OD values generated for each drug.

One plate was stained with Giemsa to examiner the cytopathic effect in the cultures.

Three separate experiments were realized. In each experiment, two culture plates were used for each compound; in each culture plate, 8 replicate wells were used for each drug concentration.

<u>Results:</u>

Similar results were obtained in the three sets of experiments. Graphic representations of the results of one representative experiment for each drug are shown on Figs. 5 a,b,c and 6 a,b,c.

<u>Nitazoxanide (Figs. 5 a,b,c):</u>

No inhibitory effect was noted for concentrations ranging between $10^{-4}$ mg/l and 0.3 mg/L. A significant effect was noted for concentration ≥0.6 mg/L, with a complete inhibition of *Toxoplasma* growth for concentrations ≥2.5 mg/L. However, a marked toxicity was noted on the cell monolayer for concentrations ≥2.5 mg/L.

Microscopic examination of the monolayer showed that NTZ, at a concentration of 1.25 mg/L induced cytopathic effect on the parasitized cells, with enlargement of the parasitophorous vacuole and reduction of the number of the intra-celular parasites. From regression analysis, the 50% inhibitory concentration could be estimated at 1.2 mg/L.

<u>Desacetyl nitazoxanide (Figs. 6 a,b,c):</u>

Similar results were obtained with desacetyl nitazoxanide: no effect for concentrations ranging between $10^{-4}$ mg/L and 0,3 mg/L, inhibition for concentration ≥0.6 mg/L, and marked toxicity for concentration ≥ 2.5 mg/L. The 50% inhibitory concentration could be estimated at 1.2 mg/L.

The results obtained were reproducible over three separate experiments, with assessment of the drug inhibitory effect on repeated cultures for each drug concentration.

For both NTZ and desacetyl NTZ, a marked inhibition of *Toxoplasma* growth could be observed at concentrations of approximately 1.2 mg/L, with alteration of the parasitophorous vacuole but no marked alteration of the parasite itself.

These results indicate that these drugs have good activity against *T. gondii,* and that a therapeutic effect can be expected in vivo based on obtaining a concentration of approximately 1 mg/L in serum or tissues.

**EXAMPLE VI**

*MYCOBACTERIA*

Nitazoxanide was found to have antimicrobial activity against TB organisms. The following table shows an assay for MIC of nitazoxanide and tizoxanide against *Mycobacterium intracellular* by agar dilution technique. These results are based upon several experiments, each of which took about 3 weeks for the agar dilution method with Middlebrook agar. The data obtained indicate that nitazoxanide has an MIC against the Mycobacteria of 2 µg/ml and tizoxanide has an MIC of 4µg/ml, using a standard strain of *Mycobacterium intracellular* from ATCC, using the standard

agar dilution assay. MICs of Nitazoxanide and tizoxanide to *Mycobacteia intracellulare*

| MIC | |
|---|---|
| Nitazoxanide<br>Tizoxanide | 2 µg/ml<br>4 µg/ml |
| *MICs were determined Middlebrook 7H11 agar 13950, a standard strain, by standard agar dilution using for 3 weeks. *M. intracellular* ATCC was used for this experiment. | |

Fig. 7 is a chart based upon the assay for nitazoxanide effectiveness against mycobacteria growing in a liquid broth. We used the MTS colorimetric assay which permits us to determine growth in 4 hours rather than 3 weeks as with the agar counting method. As can be seen from the data in Fig. 7, when nitazoxanide was added at the 72 hr after culture was initiated, there was an immediate effect on continued growth as compared to the growth in control medium alone. The 3 µg/ml dose of nitazoxanide stops growth for the next 24 hrs and then there is a slow growth afterwards for the next 2 days. The 50 µg/ml dose was completely bacteriostatic throughout the 144 hours of the culture.

**EXAMPLE VII**

*CRYPTOSPORIDIUM PARVUM*

The effect of nitazoxanide was tested against *Cryptosporidium parvum* in experimentally infected mice. Nitazoxanide was supplied by Romark Laboratories, L.C. in Tampa, Florida.

The total human dose (1 g/day for 7 days i.e. 7 g) was modified for use for mice according to Paget and Barnes. The human dose was multiplied by 0.0026 for mice (weighing approximately 20 grams) to obtain he total amount of the drug needed for each host morning and evening for 7 consecutive days. Each mouse received 2.6 mg/day (7000 mg x 0.0026/7). The doses were administered by mouth using a plastic syringe equipped with a round tip needle.

Twenty (20) 2-day old suckling mice were infected by oral administration of 100,000 oocysts of *Cryptospordium parvum* obtained from infected calves. Before being administered to mice, the oocysts were concentrated using a sugar solution according to the technique described by Fayer & Ellis. Rectal swabs from each mouse were obtained and examined daily using the modified Niehl-Neelsen staining technique described by Graczyk et al. Oocysts shedding appeared in feces 2 days after the oral infection of the animals. On the third day following infection of the animals, 10 mice received 1.3 mg of nitazoxanide, morning and evening, for 7 consecutive days while the 10 remaining mice were kept as untreated controls. Rectal swabs were obtained daily for each of the 7 days of treatment and for each of the 7 days following the end of treatment. The oocysts were suspended in oil and counted per 100 fields under a microscope.

Results:

The results shown in the following Table clearly indicate that nitazoxanide administered at a daily dose of 2.6 mg/day for 7 consecutive days was effective against *Cryptosporidium parvum* in reducing the number of oocysts in the feces of the infected mice when compared to the control animals. The test drug decreased the oocysts shedding in 6 of the 10 treated mice at the end of the third day of treatment. At the end of treatment of Day 7, there was a complete reduction of the oocyst shedding, all treatment animals having negative fecal examination when compared to untreated control mice. This effect lasted at least for 7 days after treatment as shown by negative examinations observed on days 3 and 7 after the end of treatment.

| | NO. OF OOCYST DETECTED PER OIL IMMERSION FIELD | | | | | | | |
|---|---|---|---|---|---|---|---|---|
| | At 3rd day of treatment | | At last day of treatment | | At 3rd day post-treatment | | At 7th day post-treatment | |
| Mice No. | Control group | Treated group | Control group | Treated group | Control group | Treated group | Control group | Treated group |
| 1 | 3.0 | 0.0 | 5.0 | 0.0 | 4.0 | 0.0 | 2.0 | 0.0 |
| 2 | 4.0 | 0.0 | 4.0 | 0.0 | 3.0 | 0.0 | 1.0 | 0.0 |
| 3 | 6.0 | 0.0 | 5.0 | 0.0 | 4.0 | 0.0 | 0.5 | 0.0 |
| 4 | 3.0 | 2.0 | 3.0 | 0.0 | 2.0 | 0.0 | 1.0 | 0.0 |
| 5 | 5.0 | 2.0 | 3.0 | 0.0 | 3.0 | 0.0 | 0.5 | 0.0 |
| 6 | 3.0 | 0.0 | 4.0 | 0.0 | 5.0 | 0.0 | 2.0 | 0.0 |
| 7 | 3.0 | 0.0 | 5.0 | 0.0 | 4.0 | 0.0 | 1.0 | 0.0 |
| 8 | 5.0 | 1.0 | 5.0 | 0.0 | 1.0 | 0.0 | 0.5 | 0.0 |
| 9 | 3.0 | 3.0 | 3.0 | 0.0 | 2.0 | 0.0 | 1.0 | 0.0 |
| 10 | | 0.0 | 5.0 | 0.0 | 2.0 | 0.0 | 0.5 | 0.0 |
| Total | 35 | 8.0 | 4.2 | 0.0 | 30 | 0.0 | 10 | 0.0 |
| Mean | 3.5 | 0.8 | 4.2 | 0.0 | 3.0 | 0.0 | 1.0 | 0.0 |
| Efficacy | | 60% | | 100% | | 100% | | 100% |

## EXAMPLE VIII

*MYCOBACTERIUM*

Nitazoxanide was compared against izoniazide antibiotic. The protocol used BCG (Bacille de Calmette et Guerin) as a mycobacterium strain. The sensitivity of this strain was the same as that of *M. tuberculosis*, but this strain is more harmless and thus did not require high level of containment of a tuberculosis agent.

4 mg/mouse per day in 0.2 ml of sunflower oil was administered to mice. The results in mice treated with nitazoxanide were comparable to the group receiving izoniazide.

| | $10^7$ | | | $10^5$ | | |
|---|---|---|---|---|---|---|
| | Spleen | Liver | Lungs | Spleen | Liver | Lungs |
| Nitazo | 1 575 000<br>800 000<br>875 000<br>950 000 | 1 575 000<br>1 550 000<br>1 550 000<br>750 000 | 57 500<br>122 500<br>30 000<br>75 000 | 68 250<br>65 000<br>75 000<br>60 000 | 70 000<br>87 500<br>35 000<br>60 000 | 50<br>75<br>150<br>50 |
| INH | 475 000<br>255 000<br>200 000 | 1 050 000<br>750 000<br>975 000 | 11 000<br>5 750<br>4 000 | 20 000<br>15 250<br>60 000<br>20 000 | 21 250<br>27 500<br>52 500<br>37 500 | 50<br>125<br>50<br>50 |
| PBS | 1 500 000<br>1 525 000<br>1 925 000<br>1 675 000 | 2 125 000<br>1 800 000<br>1 750 000<br>1 800 000 | 92 500<br>98 000<br>177 500<br>117 500 | 102 500<br>140 000<br>98 000<br>105 000 | 195 000<br>175 000<br>150 000<br>150 000 | 750<br>800<br>500<br>750 |

## EXAMPLE IX

*FASCIOLA HEPATICA*

The *in vitro* efficacy of nitazoxanide and desacetyl-nitazoxanide was tested against *Fasciola hepatica.*

Mature *F. hepatica* were recovered from the bile ducts of 3 calf livers condemned due to fasciolosis at the Louisiana Veterinary Medical Diagnostic Laboratory at the Hardy's Meat Packers, Bunkie, LA. Flukes were washed in sterile saline for 1 hour and transferred to sterile saline or RPMI (pH 7.4) for an additional 3 hours. Flukes were then held in sterile RPMI-rabbit serum (50:50 v/v) or sterile RPMI (pH 7.4) overnight at 37°C with 5% $CO_2$.

*In vitro* culture (37°C, 5% $CO_2$) was done according to a modification of the method of Ibarra and Jenkins (Z. Parasitenkd. 70:655-661, 1984). Using sterile technique, flukes were washed twice for 2-3 minutes in Hank's balanced salt solution (pH 7.2) and placed individually into wells of six-well Linbro culture plates containing 10 ml aliquots of the designated dilutions of the drug in culture media. The latter consisted of sterile 50:50 v/v RPMI-Rabbit serum with 2% Rabbit Blood plus 100 ppm Penicillin and 100 ppm Streptomycin. Only flukes that had normal activity and morphology were used.

Stock solutions of NTZ or its metabolite D-NTZ provided by Romark were dissolved in DMSO (2000 μg/ml) and diluted in culture medium, using 100 ml volumetric flasks, to produce the specified drug concentrations (100, 50, 25, 10, 5, 3, 1 μg/ml). Two control flukes were included in each replicate, one in unmediated culture media with RBC and one in unmedicated culture media without RBC.

Flukes were examined for the effects of drug treatment as evidenced by death, motility disturbances or morpholgic changes as compared to untreated control flukes, using a backlighted panel and a lighted 3X magnifying lens.

Results:

**Experiment 1:** For D-NTZ, flukes in the 50 and 100 μg treatments were moribund or dead within one hour. Four of 7 flukes in the 25 μg treatment were moribund, two were active, and one was sluggish within the first hour; all were dead except two sluggish flukes by three hours and only one sluggish fluke remained alive after four hours. At 10 μg, reduced activity was noted at 1, 3 and 4 hours and all were moribund or dead by 7 hours. Reduced activity in some individuals was seen by 24 hours in the 5 μg and 3 μg groups with a somewhat slower onset at 3 μg; all were dead in the 3 and 5 μg treatment wells by 50 hours except one sluggish fluke in each group. Some slowing of activity

was noted in the 1 µg group at 42-74 hours and only 3 active and one moribund fluke remained alive at 91 hours; at 115 hours only one sluggish fluke remained in the 1 /g group. Mortality in the control with RBC group was observed at 66 hours (one fluke), 91 hours (one fluke) and 115 hours (four flukes). In the Control without RBC group, all were alive at hour 91 and one was dead at hour 115.

**Experiment 2**: For NTZ, somewhat greater activity was noted by earlier effects on motility score and mortality in the 8 replicates as compared to results for D-NTZ. In the 100, 50 and 25 µg groups all flukes were dead or moribund except one fluke at 1 hour in the 25 µg group it was dead at 3 hour. Dose-related reduction in motility was seen in each of the other medicated groups beginning at hour 1. At 10 µg, only one fluke survived to 16 hours. In the 5 µg group, only 3 flukes were active at hour 6 and none were active at 16 hours. By 23 hours, only 2 sluggish flukes in the 3 µg group remained alive; these were dead by hour 41. For the 1 µg group, one fluke died by hour 16, three by hour 41 and five by hour 74; 3 flukes remained active at hour 91 and one fluke had activity at hour 115. In the Control with RBC group, 7 of 8 flukes were alive at hour 74, 3 were alive at hour 91 and 2 survived at hour 115. In the Control without RBC group, 6 of 8 flukes had activity at hour 74, 4 were active at hour 91 and two remained active at hour 115.

Fluke death in the high dose groups (25, 50, 100 µg) was rapid and associated with contraction and ventral 'curling'. At lower medication levels, most flukes slowed for a period and were more relaxed and 'flattened' when moribund or dead.

Contamination became limiting on experimental results in some replicates beginning at hour 91. For the D-NTZ experiment, major bacterial or fungal overgrowth and associated mortality in two replicate plates occurred by hour 115. For the NTZ experiment, overgrowth and fluke mortality in entire replicate plates occurred by hour 91 (two replicates), and hour 115 (5 replicates). Hour 139 observations were not considered valid because of general contamination of most plates.

Conclusions:

Strong flukecidal efficacy by nitazoxanide is suggested by experiments with both drugs tested. Somewhat greater flukecidal activity against *F. hepatica* was observed for nitaxoxanide than for desacetyl-nitazoxanide, the main metabolite, which is thought to be active at the hepatic level.

Rapid fluke death occurs within 1 hour at *in vitro* D-NTZ medication rates of >50 µg, within 4 hours at 25 µg and by 6-7 hours at 10µg. Ten µg may be an appropriate single treatment target drug delivery rate if pharmacokinetic data indicate tissue levels are maintained for >6-8 hours after a single treatment.

Strong flukecidal activity by 74 hours (three days) was observed for both compounds at the 3 and 5 µg dose rates. Prolonged survival approaching, but not equal to unmedicated control flukes was observed at the 1µg dose level; delivery of this level of drug to flukes in hepatic tissues for 3 to 4 days may therefore have inadequate therapeutic effect on parasites.

## EXAMPLE X

*FASCIOLA GIGANTICA*

Nitazoxanide was tested against immature and mature *Fasciola gigantica in* experimentally infected rabbits.

*Fasciola gigantica* encysted metacercariae (EMC) were collected on cellophane sheet after 28-35 days from infection of *L. calludi* snails by *Fasciola gigantica* miracidium using the technique described by Abdel-Ghany where snails were exposed daily to artificial light, for 30 minutes, in clean dechlorinated tap water. The resulting encysted metacercaraie (EMC) were preserved at 4°C in a refrigerator for 5 to 8 days under the surface of water until they were used to infect experimental animals.

Forty (40) Boscat rabbits, weighting 1.5 to 2 kg each, were included in the study and allocated to two treatment groups of 20 animals.

Animals from Group 1 were orally infected with 35-40 encysted metacercarae wrapped in a leaf of lettuce and pushed on the root of the tongue of the animals. The mouths of the animals were held closed by hand until the encysted metacercariae were swallowed. These Group 1 animals were used to test the efficacy of nitazoxanide against immature stages (4-5 weeks old) of *Fasciola gigantica.*

Animals from Group 2 were orally infected as indicated above with 10-15 encysted metacercariae and were used to test the efficacy of nitazoxanide against the early mature flukes (>10 weeks old).

Ten animals from Group 1 received 35 mg of nitazoxanide, morning and evening, for 7 consecutive days 4 weeks after their infection at the immature stage of the parasite cycle. The ten remaining animals in Group 1 were kept as untreated controls.

Ten animals from Group 2 received 35 mg of nitazoxanide, morning and evening, for 7 consecutive days 10 weeks after their infection at the mature stage of the parasite. The 10 remaining animals in Group 2 were kept as

untreated controls.

All animals were fed with dry ration until the end of the experiment.

Seven days after administration of the last dose of nitazoxanide, all rabbits from each group were sacrificed. The surface of the liver was examined for the presence of necrotic migrating furrows especially at the immature stage of the parasite cycle. These necrotic areas were examined using two surgical needles in order to extract the juvenile migrating flukes according to the technique described by El-Bahy. The livers were sliced in small pieces especially around the migrating furrows and macerated under a microscope in order to extract the existing flukes. The abdominal cavity and the visceral surfaces were washed with warm water. The water was then collected, sieved and examined for identification of juvenile flukes. All the collected parasites as well as parts of them were counted in both treated and untreated animal in both Groups 1 and 2. Living flukes appeared pink in color, translucid showing intact teguments, easily extractable from the tissue of the livers using warm water, while dead flukes were grayish in color, loose and showed a broken necrotic surface. The efficacy of nitazoxanide was calculated by using the formula indicated below:

$$\% \text{ efficacy} = \frac{a - b}{a} \times 100$$

Where:

a = the number of flukes recovered from feces in the control animals
b = the number of flukes recovered from feces in the treated animals.

**Results**

The results of the study as indicated in Table 7 show a marked decrease in the number of immature flukes recovered from the liver of rabbits in the treated group when compared in the control group. The means percentage of reduction was calculated as 46.77% (range: 40-60%).

Table 7:

| Efficacy of nitazoxanide against immature - (4-week/old) F. gigantica in experimentally infected rabbits | | | |
|---|---|---|---|
| **No. of flukes extracted from liver of** | | | |
| **Rabbit No.** | **Untreated Control** | **Treated Rabbits** | **Efficacy %** |
| 1 | 7 | 4 | 42% |
| 2 | 7 | 4 | 42% |
| 3 | 6 | 3 | 50% |
| 4 | 8 | 4 | 50% |
| 5 | 5 | 3 | 40% |
| 6 | 5 | 2 | 60% |
| 7 | 5 | 3 | 40% |
| 8 | 6 | 3 | 50% |
| 9 | 8 | 4 | 50% |
| 10 | 5 | 3 | 40% |
| Mean | 6.2 | 3.3 | 46.77% |

At the early mature stage of their infection, nitazoxanide showed a complete effect (100% reduction) and no worms could be seen after examination of the liver of the treated rabbits in comparison with the untreated control animals as shown in Table 8.

Table 8:

| Efficacy of nitazoxanide against early mature (10-week/old) F. gigantica in experimentally infected rabbits | | | |
|---|---|---|---|
| **No. of flukes extracted from liver of** | | | |
| **Rabbit No.** | **Untreated Control** | **Treated Rabbits** | **Efficacy %** |
| 1 | 4 | 0.0 | 100% |
| 2 | 4 | 0.0 | 100% |
| 3 | 3 | 0.0 | 100% |
| 4 | 3 | 0.0 | 100% |
| 5 | 2 | 0.0 | 100% |
| 6 | 2 | 0.0 | 100% |
| 7 | 2 | 0.0 | 100% |
| 8 | 3 | 0.0 | 100% |
| 9 | 3 | 0.0 | 100% |
| 10 | 3 | 0.0 | 100% |
| Mean | 2.9 | 0.0 | 100% |

Nitazoxanide administered as a 70 mg/day dose for 7 consecutive days is moderately effective against immature stage of *Fasciola gigantica* and completely effective against the early mature stage of the parasite.

**EXAMPLE XIII**

*SCHISTOSOMA*

Nitazoxanide was tested against *Schistosoma mansoni* and *Schistosoma hematobium* in experimentally infected mice.

Forty (40) white mice, weighing 30 to 50 grams were allocated to two treatment groups of 20 animal per group. The first group was infected with 300-500 *Schistosoma mansoni* free active cercariae suspended in 0.25 ml of distilled water and administered to each mouse by intraperitoneal injection. The second group was infected in the same manner but with *Schistosoma hemotobium* cercariae. These two groups were then kept for a total of 70 days in the laboratory.

Seventy days after infection of the animals, ten mice from each group were treated with nitazoxanide as a 1.3 mg oral dose administered, morning and evening, for 7 consecutive days. Seven days after the end of treatment tall mice were sacrificed and the worms were extracted from the liver of each animal by perfusion using tepid water (37°C). The extracted schistosomes were counted for all treatment and control animals. The efficacy of nitazoxanide was calculated busing the formula indicated below:

$$\% \text{ efficacy} = \frac{a - b}{a} \times 100$$

Where:

a = the number of schistosomes recovered from feces in the control animals
b = the number of schistosomes recovered from feces in the treated animals

**Results**

The results shown in Tables 9 and 10 clearly indicate that nitazoxanide administered at a daily dose of 2.6 mg/day for 7 consecutive days was more effective against *Schistosoma hematobium* where a worm reduction of 82.85% was observed when compared to the control animals while against *Schistosoma mansoni,* the worm reduction only achieved 59.91% versus the control mice. These results are consistent with those report by Abaza et al. in patients where nitazoxanide was not effective against *S. mansoni* as shown by nitazoxanide posttreatment positive egg-counts.

Table 9:

| Efficacy of nitazoxanide against mature (13-week/old) Schistosoma mansoni in mice | | |
|---|---|---|
| **No. of flukes removed from liver of** | | |
| **Mice No.** | **Untreated Control** | **Treated Mice** |
| 1 | 21 | 10 |
| 2 | 29 | 9 |
| 3 | 32 | 10 |
| 4 | 26 | 11 |
| 5 | 24 | 13 |
| 6 | 19 | 10 |
| 7 | 20 | 9 |
| 8 | 24 | 12 |
| 9 | 22 | 8 |
| 10 | 30 | 7 |
| Total | 247 | 99 |
| Mean/Mouse | 24.7 | 9.9 |
| Efficacy | | 59.91 |

Table 10:

| Efficacy of nitazoxanide against mature (13-week/old) Schistosoma hematobium in mice | | |
|---|---|---|
| **No. of flukes removed from liver of** | | |
| **Mice No.** | **Untreated Control** | **Treated Mice** |
| 1 | 18 | 3 |
| 2 | 16 | 3 |
| 3 | 14 | 2 |
| 4 | 19 | 2 |
| 5 | 12 | 4 |
| 6 | 10 | 4 |
| 7 | 13 | 2 |
| 8 | 12 | 2 |
| 9 | 17 | 0.0 |
| 10 | 9 | 2 |
| Total | 140 | 24 |
| Mean/Mouse | 14 | 2.4 |
| Efficacy | | 82.85 |

## Claims

**1.** An oral pharmaceutical composition containing as active agent at least one compound selected from the group consisting of:

compound of formula (I)

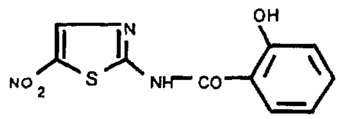

(I)

and compound of formula (II)

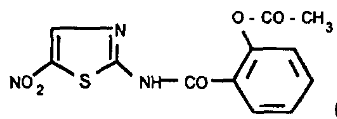

(II)

wherein said active agent is in the form of active particles having a particulate size smaller than 200 µm and a mean particle size of greater than 10 µm.

2. The composition of Claim 1, in which the mean particle size of the said active solid particles is between 10 and 100 µm.

3. The composition of Claim 1, in which the mean particle size of the said active solid particles is between 20 and 50 µm.

4. The composition of Claim 1, in which less than 10% by weight of the said active solid particles has a particle size larger than 100 µm.

5. The composition of Claim 1, in which at least 50% by weight of the said active solid particles has a particle size smaller than 50 µm.

6. The composition of Claim 1, in which less than 10% by weight of the said active solid particles has a particle size smaller than 5 µm.

7. The composition of Claim 1 in which the distribution factor of the said active solid particles is between 0.8 and 2, said distribution factor being calculated by the following formula:

$$F_{90\%} = (\Phi_{90\%} -. \Phi_{10\%}) / ((.\Phi_{90\%} + \Phi_{10\%})/2)$$

in which

$F_{90\%}$ is the distribution factor at 90%;

$\Phi_{90\%}$ is the maximum particle size of the fraction of particles corresponding to 90% of the said active solid particles, and

$\Phi_{10\%}$ is the maximum particle size of the fraction of particles corresponding to 10% of the said active solid particles.

8. The composition of Claim 1, in which the distribution factor of the said active solid particles is between 1.1 and 1.9, said distribution factor being calculated by the following formula

$$F_{90\%} = (\Phi_{90\%} -. \Phi_{10\%}) / ((. \Phi_{90\%} + \Phi_{10\%})/2)$$

in which

$F_{90\%}$ is the distribution factor at 90%;

$\Phi_{90\%}$ is the maximum particle size of the fraction of particles corresponding to 90% of the said active solid particles, and

$\Phi_{10\%}$ is the maximum particle size of the fraction of particles corresponding to 10% of the said active solid particles.

**9.** An oral pharmaceutical composition containing as active agent at least one compound selected from the group consisting of:

compound of formula (I)

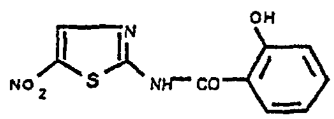

(I)

and compound of formula (II)

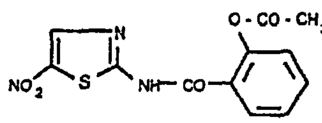

(II)

and a stability improving amount of a pharmaceutically acceptable acid.

**10.** The composition of Claim 9, in which the pharmaceutically acceptable acid is selected from the group consisting of citric acid, glutamic acid, succinic acid, ethanesulfonic acid, acetic acid, tartaric acid, ascorbic acid, methanesulfonic acid, fumaric acid, adipic acid, malic acid, and mixtures thereof.

**11.** The composition of Claim 9, in which the ratio of the weight of pharmaceutically acceptable acid/the weight of said active solid particles, is between 0.01 and 0.5.

**12.** The composition of Claim 9, in which the ratio of the weight of pharmaceutically acceptable acid/the weight of said active solid particles is between 0.03 and 0.2.

**13.** Use of at least one compound selected from the group consisting of a compound of formula (I):

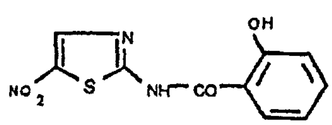

(I)

and a compound of formula (II):

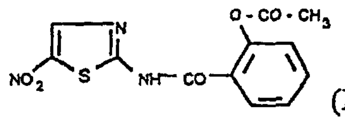

(II)

as active agent for manufacture of a pharmaceutical composition for treating an infection in an immunocompromised mammal by a microorganism selected form the group consisting of Cryptosporidium parvum, Isospora belli, Enterocytzoon bieneusi, Encephalitozoon intestinalis, Mycobacterium tuberculosis, Mycobacterium avium intracellulare, Pneumocystis carinii, and Toxoplasma gondii wherein said active agent is in the form of active particles having a particle size smaller than 200 µm and a mean particle size of greater than 10 µm.

**14.** A use as in claim 13, wherein said active agent is in the form of particles with a mean particle size of between 20 and 50 µm.

**15.** A use as in claim 13, wherein said pharmaceutical composition contains at least one pharmaceutically acceptable acid.

**16.** A use as in claim 15, wherein said pharmaceutically acceptable acid is selected from the group consisting of citric acid, glutamic acid, succinic acid, ethanesulfonic acid, acetic acid, tartaric acid, ascorbic acid, methanesulfonic acid, fumaric acid, adipic acid, malic acid and mixtures thereof.

**17.** A use as in claim 13, wherein said active agent is a compound of formula (I).

**18.** A use as in claim 13, wherein said active agent is a compound of formula (II).

**19.** A use as in claim 13, wherein said mammal is human and wherein said active agent is administered in an amount of from 500-2000 mg daily.

**20.** Use of at least one compound selected from the group consisting of a compound of formula (I):

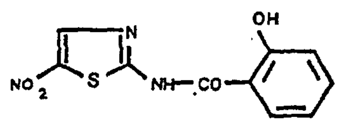

(I)

and a compound of formula (II):

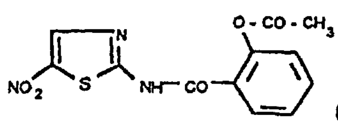

(II)

as active agent for manufacture of a pharmaceutical composition for treating a parasitic infection by a trematode wherein said active agent is in the form of active particles having a particle size smaller than 200 µm and a mean particle size greater than 10 µm.

**21.** A use as in claim 20, wherein the trematode is selected from the group consisting of Schistosoma Fasciola, Fasciolopsis, Dicrocoelium, Heterophyes, and Matagonimus.

**22.** A use as in claim 21, wherein the trematode is selected from the group consisting of Schistosoma mansoni, Schistosoma haematobium, Schistosoma mekongi, Schistosoma japonicum, Schistosoma intercalatum, Fasciola hepatica, Fasciola gigantica, Fasciolopsis biski, Dicrocoelium dendriticum, Heterophyes heterophyes, and Metagonimus yokogawa.

**23.** A use as in claim 21, wherein said active agent is in the form of particles with a mean particle size of between 20 and 50 μm.

**24.** A use as in claim 21, wherein said pharmaceutical composition contains at least one pharmaceutically acceptable acid.

**25.** A use as in claim 24, wherein said pharmaceutically acceptable acid is selected from the group consisting of citric acid, glutamic acid, succinic acid, ethanesulfonic acid, acetic acid, tartaric acid, ascorbic acid, methanesulfonic acid, fumaric acid, adipic acid, malic acid and mixtures thereof.

**26.** A use as in claim 21, wherein the ratio of the weight of pharmaceutically acceptable acid/the weight of said active particles is between 0.01 and 0.5.

**27.** A use as in claim 21, wherein said active agent is a compound of formula (I).

**28.** A use as in claim 21, wherein said active agent is a compound of formula (II).

**29.** A pharmaceutical paste for topical administration, said paste comprising:

as active agent, solid particles of at least one compound selected from the group consisting of:

compound of formula (I)

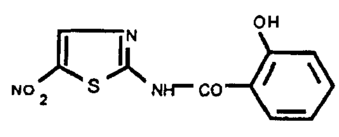

(I)

and compound of formula (II)

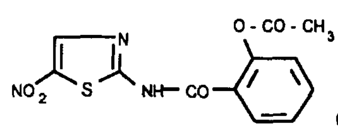

(II)

said particles having a particle size smaller than 200 μm and a mean particle size greater than 10 μm;

at least one thickener;

at least one wetting agent; and

at least one pharmaceutically acceptable acid, the pH of the paste being between 2 and 6.

**30.** A pharmaceutical paste as in Claim 29, wherein said paste further comprises at least one additive selected from the group consisting of cetylic alcohol, glyceride derivatives, proyplene glycol, and mixtures thereof.

**31.** A pharmaceutical composition for oral administration containing active agent granulated in the presence of a granulating agent, wherein:

said active agent is in the form of solid active particles of at least one compound selected from the group consisting of:

compound of formula (I)

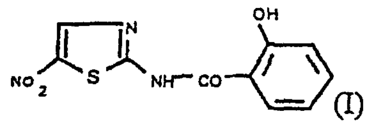
(I)

and compound of formula (II)

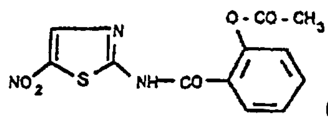
(II)

and wherein said active particles have a particle size smaller than 200 µm and a mean particle size of greater than 10 µm.

**32.** The composition of Claim 31, wherein said granulating agent is selected from the group consisting of polyvinylpyrrolidone, water, alcohol, sucrose, hydroxyl cellulose, and mixtures thereof.

**33.** The composition of Claim 31, wherein said granulated active solid particles contain at least one pharmaceutically acceptable acid.

**34.** The composition of Claim 33, wherein said pharmaceutically acceptable acid is selected from the group consisting of citric acid, glutamic acid, succinic acid, ethanesulfonic acid, acetic acid, tartaric acid, ascorbic acid, methanesulfonic acid, fumaric acid, adipic acid, malic acid, and mixtures thereof.

**35.** The composition of Claim 33, wherein the ratio of the weight of pharmaceutically acceptable acid/the weight of said active agent is between 0.01 and 0.5.

**36.** A pharmaceutical composition for oral administration containing active agent, a wetting agent and a starch derivative, wherein:

said active agent is in the form of solid active particles of at least one compound selected from the group consisting of:

compound of formula (I)

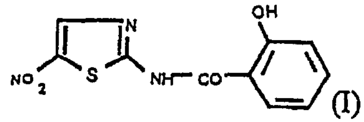
(I)

and compound of formula (II)

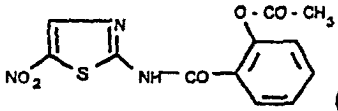

(II)

and wherein said active particles having a particle size smaller than 200 µm and a mean particle size of greater than 10 µm.

**37.** The pharmaceutical composition of Claim 36, further comprising at least one pharmaceutically acceptable acid.

**38.** The pharmaceutical composition of Claim 36, wherein the active particles are granulated in the presence of a granulating agent to form granulated active agent containing from 2 to 99.97% by weight of said active compound and from 0.03 to 10% by weight of a granulating agent.

**39.** The pharmaceutical composition of Claim 38, wherein said granulating agent is selected among the group consisting of polyvinylpyrrolidone, water, alcohol, sucrose, hydroxyl cellulose and mixtures thereof.

**40.** A liquid suspension of active agent for oral administration, said suspension containing:

as active agent, solid particles of at least one compound selected from the group consisting of:

compound of formula (I)

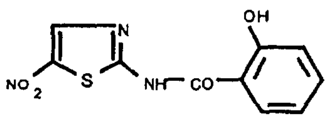

(I)

and compound of formula (II)

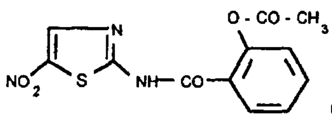

(II)

wherein said active agent is in the form of active particles having a particle size smaller than 200 µm and a mean particle size greater than 10 µm; and
at least one pharmaceutically acceptable acid, the pH of the suspension being between 2 and 6.

**41.** The suspension of Claim 40, wherein the pH of the suspension is between 3 and 5.

**42.** The suspension of Claim 40, further comprising a granulating agent.

**Patentansprüche**

**1.** Orale pharmazeutische Zusammensetzung, die als Wirkstoff wenigstens eine Verbindung enthält, die ausgewählt ist aus der Gruppe, die aus:

Verbindung mit Formel (I)

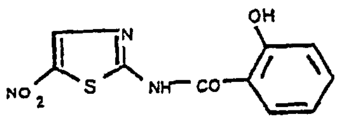

(I)

und Verbindung mit Formel (II)

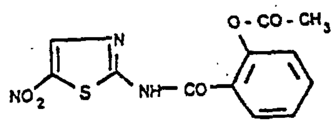

(II)

besteht, wobei besagter Wirkstoff in der Form aktiver Teilchen mit einer Teilchengröße von weniger als 200 µm und einer mittleren Teilchengröße von mehr als 10 µm vorliegt.

**2.** Zusammensetzung nach Anspruch 1, **dadurch gekennzeichnet, daß** die mittlere Teilchengröße der besagten aktiven festen Teilchen zwischen 10 und 100 µm liegt.

**3.** Zusammensetzung nach Anspruch 1, **dadurch gekennzeichnet, daß** die mittlere Teilchengröße der besagten aktiven festen Teilchen zwischen 20 und 50 µm liegt.

**4.** Zusammensetzung nach Anspruch 1, **dadurch gekennzeichnet, daß** weniger als 10 Gew.-% der besagten aktiven festen Teilchen eine Teilchengröße von mehr als 100 µm besitzen.

**5.** Zusammensetzung nach Anspruch 1, **dadurch gekennzeichnet, daß** wenigstens 50 Gew.-% der besagten aktiven festen Teilchen eine Teilchengröße von weniger als 50 µm besitzen.

**6.** Zusammensetzung nach Anspruch 1, **dadurch gekennzeichnet, daß** weniger als 10 Gew.-% der besagten aktiven festen Teilchen eine Teilchengröße von weniger als 5 µm besitzen.

**7.** Zusammensetzung nach Anspruch 1, **dadurch gekennzeichnet, daß** der Verteilungsfaktor der besagten aktiven festen Teilchen zwischen 0,8 und 2 liegt, wobei besagter Verteilungsfaktor berechnet wird mit der folgenden Formel:

$$F_{90\%} = (\Phi_{90\%} - \Phi_{10\%}) / ((\Phi_{90\%} + \Phi_{10}\%)/2)$$

in der

$F_{90\%}$ der Verteilungsfaktor bei 90 % ist;

$\Phi_{90\%}$ die maximale Teilchengröße der Fraktion von Teilchen ist, die 90 % der besagten aktiven festen Teilchen entspricht, und

$\Phi_{10\%}$ die maximale Teilchengröße der Fraktion von Teilchen ist, die 10 % der besagten aktiven festen Teilchen entspricht.

**8.** Zusammensetzung nach Anspruch 1, **dadurch gekennzeichnet, daß** der Verteilungsfaktor der besagten aktiven festen Teilchen zwischen 1,1 und 1,9 liegt, wobei besagter Verteilungsfaktor berechnet wird mit der folgenden Formel

$$F_{90\%} = (\Phi_{90\%} - \Phi_{10\%})/((\Phi_{90\%} + \Phi_{10\%})/2)$$

in der

$F_{90\%}$ der Verteilungsfaktor bei 90 % ist;

$\Phi_{90\%}$ die maximale Teilchengröße der Fraktion von Teilchen ist, die 90 % der besagten aktiven festen Teilchen entspricht, und

$\Phi_{10\%}$ die maximale Teilchengröße der Fraktion von Teilchen ist, die 10 % der besagten aktiven festen Teilchen entspricht.

**9.** Orale pharmazeutische Zusammensetzung, die als Wirkstoff wenigstens eine Verbindung enthält, die ausgewählt ist aus der Gruppe, die aus:

Verbindung mit Formel (I)

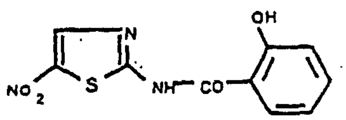

(I)

und Verbindung mit Formel (II)

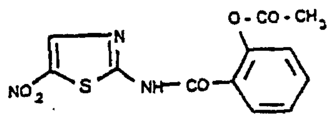

(II)

besteht, und eine die Stabilität verbessernde Menge einer pharmazeutisch annehmbaren Säure.

**10.** Zusammensetzung nach Anspruch 9, **dadurch gekennzeichnet, daß** die pharmazeutisch annehmbare Säure ausgewählt ist aus der Gruppe, die aus Zitronensäure, Glutaminsäure, Bernsteinsäure, Ethansulfonsäure, Essigsäure, Weinsäure, Ascorbinsäure, Methansulfonsäure, Fumarsäure, Adipinsäure, Äpfelsäure und Mischungen derselben besteht.

**11.** Zusammensetzung nach Anspruch 9, **dadurch gekennzeichnet, daß** das Verhältnis Gewicht der pharmazeutisch annehmbaren Säure/Gewicht besagter aktiver fester Teilchen zwischen 0,01 und 0,5 liegt.

**12.** Zusammensetzung nach Anspruch 9, **dadurch gekennzeichnet, daß** das Verhältnis Gewicht der pharmazeutisch annehmbaren Säure/Gewicht besagter aktiver fester Teilchen zwischen 0,03 und 0,2 liegt.

**13.** Verwendung wenigstens einer Verbindung, die ausgewählt ist aus der Gruppe, die aus einer Verbindung mit Formel (I):

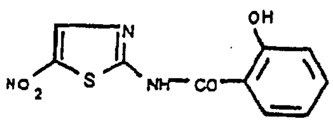

(I)

und einer Verbindung mit Formel (II):

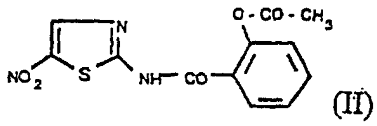

(II)

besteht, als Wirkstoff zur Herstellung einer pharmazeutischen Zusammensetzung zur Behandlung einer Infektion in einem Säuger mit beeinträchtigtem Immunsystem durch einen Mikroorganismus, der ausgewählt ist aus der Gruppe, die aus Cryptosporidium parvum, Isospora belli, Enterocytzoon bieneusi, Encephalitozoon intestinalis, Mycobacterium tuberculosis, Mycobacterium avium intracellulare, Pneumocystis carinii, und Toxoplasma gondii besteht, wobei besagter Wirkstoff in der Form von aktiven Teilchen mit einer Teilchengröße von weniger als 200 µm und einer mittleren Teilchengröße von mehr als 10 µm vorliegt.

**14.** Verwendung nach Anspruch 13, **dadurch gekennzeichnet, daß** besagter Wirkstoff in der Form von Teilchen mit einer mittleren Teilchengröße von zwischen 20 und 50 µm vorliegt.

**15.** Verwendung nach Anspruch 13, **dadurch gekennzeichnet, daß** besagte pharmazeutische Zusammensetzung wenigstens eine pharmazeutisch annehmbare Säure enthält.

**16.** Verwendung nach Anspruch 15, **dadurch gekennzeichnet, daß** besagte pharmazeutisch annehmbare Säure ausgewählt ist aus der Gruppe, die aus Zitronensäure, Glutaminsäure, Bernsteinsäure, Ethansulfonsäure, Essigsäure, Weinsäure, Ascorbinsäure, Methansulfonsäure, Fumarsäure, Adipinsäure, Äpfelsäure und Mischungen derselben besteht.

**17.** Verwendung nach Anspruch 13, **dadurch gekennzeichnet, daß** besagter Wirkstoff eine Verbindung mit Formel (I) ist.

**18.** Verwendung nach Anspruch 13, **dadurch gekennzeichnet, daß** besagter Wirkstoff eine Verbindung mit Formel (II) ist.

**19.** Verwendung nach Anspruch 13, **dadurch gekennzeichnet, daß** besagter Säuger menschlich ist und daß besagter Wirkstoff in einer Menge von 500 bis 2.000 mg täglich verabreicht wird.

**20.** Verwendung wenigstens einer Verbindung, die ausgewählt ist aus der Gruppe, die aus einer Verbindung mit Formel (I):

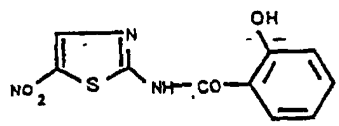

(I)

und einer Verbindung mit Formel (II):

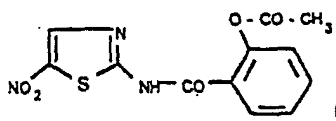

(II)

besteht, als Wirkstoff zur Herstellung einer pharmazeutischen Zusammensetzung zur Behandlung einer Parasiteninfektion durch eine Trematode, wobei besagter Wirkstoff in der Form von aktiven Teilchen mit einer Teilchengröße von weniger als 200 µm und einer mittleren Teilchengröße von mehr als 10 µm vorliegt.

**21.** Verwendung nach Anspruch 20, **dadurch gekennzeichnet, daß** die Trematode ausgewählt ist aus der Gruppe, die aus Schistosoma, Fasciola, Fasciolopsis, Dicrocoelium, Heterophyes und Matagonimus besteht.

**22.** Verwendung nach Anspruch 21, **dadurch gekennzeichnet, daß** die Trematode ausgewählt ist aus der Gruppe, die aus Schistosoma mansoni, Schistosoma haematobium, Schistosoma mekongi, Schistosoma japonicum, Schistosoma intercalatum, Fasciola hepatica, Fasciola gigantica, Fasciolopsis biski, Dicrocoelium dendriticum, Heterophyes heterophyes und Metagonismus yokogawa besteht.

**23.** Verwendung nach Anspruch 21, **dadurch gekennzeichnet, daß** besagter Wirkstoff in der Form von Teilchen mit einer mittleren Teilchengröße von zwischen 20 und 50 µm vorliegt.

**24.** Verwendung nach Anspruch 21, **dadurch gekennzeichnet, daß** besagte pharmazeutische Zusammensetzung wenigstens eine pharmazeutisch annehmbare Säure enthält.

**25.** Verwendung nach Anspruch 24, **dadurch gekennzeichnet, daß** besagte pharmazeutisch annehmbare Säure ausgewählt ist aus der Gruppe, die aus Zitronensäure, Glutaminsäure, Bernsteinsäure, Ethansulfonsäure, Essigsäure, Weinsäure, Ascorbinsäuie, Methansulfonsäure, Fumarsäure, Adipinsäure, Äpfelsäure und Mischungen derselben besteht.

**26.** Verwendung nach Anspruch 21, **dadurch gekennzeichnet, daß** das Verhältnis Gewicht der pharmazeutisch annehmbaren Säure/Gewicht besagter aktiven Teilchen zwischen 0,01 und 0,5 liegt.

**27.** Verwendung nach Anspruch 21, **dadurch gekennzeichnet, daß** besagter Wirkstoff eine Verbindung mit Formel (I) ist.

**28.** Verwendung nach Anspruch 21, **dadurch gekennzeichnet, daß** besagter Wirkstoff eine Verbindung mit Formel (II) ist.

**29.** Pharmazeutische Paste zur topischen Verabreichung, wobei besagte Paste umfaßt:

als Wirkstoff, feste Teilchen wenigstens einer Verbindung, die ausgewählt ist aus der Gruppe, die aus:

Verbindung mit Formel (I)

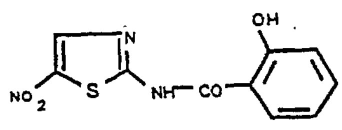
(I)

und Verbindung mit Formel (II)

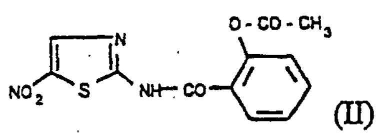
(II)

besteht, wobei besagte Teilchen eine Teilchengröße von weniger als 200 µm und eine mittlere Teilchengröße von mehr als 10 µm aufweisen;

wenigstens ein Verdickungsmittel;

wenigstens ein Benetzungsmittel; und

wenigstens eine pharmazeutisch annehmbare Säure, wobei der pH der Paste zwischen 2 und 6 liegt.

**30.** Pharmazeutische Paste nach Anspruch 29, **dadurch gekennzeichnet, daß** besagte Paste weiter wenigstens

einen Zusatzstoff enthält, der ausgewählt ist aus der Gruppe die aus Cetylalkohol, Glycerid-Derivaten, Propylenglykol und Mischungen derselben besteht.

**31.** Pharmazeutische Zusammensetzung zur oralen Verabreichung, die Wirkstoff enthält, der in Gegenwart eines Granulierungsmittels granuliert worden ist, wobei:

besagter Wirkstoff in der Form von festen aktiven Teilchen wenigstens einer Verbindung vorliegt, die ausgewählt ist aus der Gruppe, die aus:

Verbindung mit Formel (I)

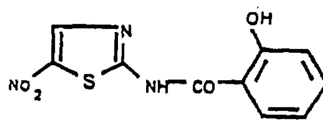

(I)

und Verbindung mit Formel (II)

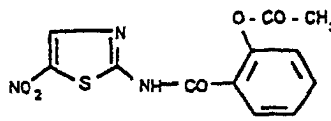

(II)

besteht, und wobei besagte aktive Teilchen eine Teilchengröße von weniger als 200 µm und eine mittlere Teilchengröße von mehr als 10 µm aufweisen.

**32.** Zusammensetzung nach Anspruch 31, **dadurch gekennzeichnet, daß** besagtes Granulierungsmittel ausgewählt ist aus der Gruppe, die aus Polyvinylpyrrolidon, Wasser, Alkohol, Saccharose, Hydroxylcellulose und Mischungen derselben besteht.

**33.** Zusammensetzung nach Anspruch 31, **dadurch gekennzeichnet, daß** besagte granulierte aktive feste Teilchen wenigstens eine pharmazeutisch annehmbare Säure enthalten.

**34.** Zusammensetzung nach Anspruch 33, **dadurch gekennzeichnet, daß** besagte pharmazeutische annehmbare Säure ausgewählt ist aus der Gruppe, die aus Zitronensäure, Glutaminsäure, Bernsteinsäure, Ethansulfonsäure, Essigsäure, Weinsäure, Ascorbinsäure, Methansulfonsäure, Fumarsäure, Adipinsäure, Äpfelsäure und Mischungen derselben besteht.

**35.** Zusammensetzung nach Anspruch 33, **dadurch gekennzeichnet, daß** das Verhältnis Gewicht der pharmazeutisch annehmbaren Säure/Gewicht besagten Wirkstoffes zwischen 0,01 und 0,5 liegt.

**36.** Pharmazeutische Zusammensetzung zur oralen Verabreichung, die Wirkstoff, ein Benetzungsmittel und ein Stärke-Derivat enthält, wobei:

besagter Wirkstoff in der Form von festen aktiven Teilchen wenigstens einer Verbindung vorliegt, die ausgewählt ist aus der Gruppe, die aus:

Verbindung mit Formel (I)

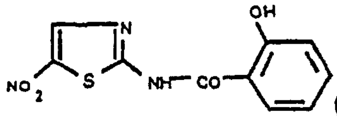

(I)

und Verbindung mit Formel (II)

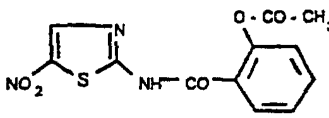

(II)

besteht, und wobei besagte aktive Teilchen eine Teilchengröße von weniger als 200 µm und eine mittlere Teilchengröße von mehr als 10 µm aufweisen.

**37.** Pharmazeutische Zusammensetzung nach Anspruch 36, **dadurch gekennzeichnet, daß** sie weiter wenigstens eine pharmazeutisch annehmbare Säure enthält.

**38.** Pharmazeutische Zusammensetzung nach Anspruch 36, **dadurch gekennzeichnet, daß** die aktiven Teilchen in Gegenwart eines Granulierungsmittels granuliert worden sind, um granulierten Wirkstoff zu bilden, der von 2 bis 99,97 Gew.-% besagter aktiven Verbindung und von 0,03 bis 10 Gew.-% eines Granulierungsmittels enthält.

**39.** Pharmazeutische Zusammensetzung nach Anspruch 38, **dadurch gekennzeichnet, daß** besagtes Granulierungsmittel ausgewählt ist aus der Gruppe, die aus Polyvinylpyrrolidon, Wasser, Alkohol, Saccharose, Hydroxylcellulose und Mischungen derselben besteht.

**40.** Flüssige Suspension von Wirkstoff zur oralen Verabreichung, wobei besagte Suspension enthält:

als Wirkstoff, feste Teilchen wenigstens einer Verbindung, die ausgewählt ist aus der Gruppe, die aus:

Verbindung mit Formel (I)

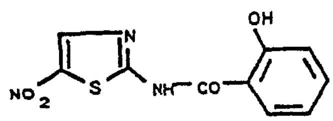

(I)

und Verbindung mit Formel (II)

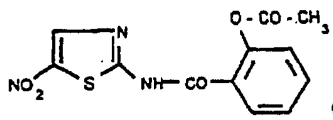

(II)

besteht, wobei besagter Wirkstoff in der Form von aktiven Teilchen mit einer Teilchengröße von weniger als 200 µm und einer mittleren Teilchengröße von mehr als 10 µm vorliegt; und
wenigstens eine pharmazeutisch annehmbare Säure, wobei der pH der Suspension zwischen 2 und 6 liegt.

**41.** Suspension nach Anspruch 40, **dadurch gekennzeichnet, daß** der pH der Suspension zwischen 3 und 5 liegt.

**42.** Suspension nach Anspruch 40, **dadurch gekennzeichnet, daß** sie weiter ein Granulierungsmittel umfaßt.

## Revendications

1. Composition pharmaceutique orale contenant en tant qu'agent actif au moins un composé choisi dans le groupe constitué par :

   un composé de formule (I)

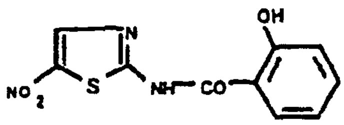

(I)

   et un composé de formule (II)

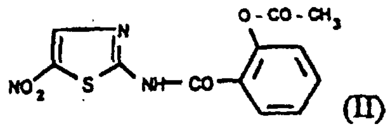

(II)

   dans laquelle ledit agent actif est sous forme de particules actives ayant une granulométrie inférieure à 200 µm et une granulométrie moyenne supérieure à 10 µm.

2. Composition selon la revendication 1, dans laquelle la granulométrie moyenne desdites particules solides actives est comprise entre 10 et 100 µm.

3. Composition selon la revendication 1, dans laquelle la granulométrie moyenne desdites particules solides actives est comprise entre 20 et 50 µm.

4. Composition selon la revendication 1, dans laquelle moins de 10 % en poids desdites particules solides actives ont une granulométrie supérieure à 100 µm.

5. Composition selon la revendication 1, dans laquelle au moins 50 % en poids desdites particules solides actives ont une granulométrie inférieure à 50 µm.

6. Composition selon la revendication 1, dans laquelle moins 10 % en poids desdites particules solides actives ont une granulométrie inférieure à 5 µm.

7. Composition selon la revendication 1, dans laquelle le facteur de distribution desdites particules solides actives est compris entre 0,8 et 2, ledit facteur de distribution étant calculé par la formule suivante :

$$F_{90\%} = (\phi_{90\%} - \phi_{10\%})/((\phi_{90\%} + \phi_{10\%})/2)$$

   dans laquelle $F_{90}$ est le facteur de distribution à 90 % ;

   $\phi_{90\%}$ est la granulométrie maximale de la fraction de particules correspondant à 90 % desdites particules solides actives, et
   $\phi_{10\%}$ est la granulométrie maximale de la fraction de particules correspondant à 10 % desdites particules solides actives.

8. Composition selon la revendication 1, dans laquelle le facteur de distribution desdites particules solides actives est compris entre 1,1 et 1,9, ledit facteur de distribution étant calculé par la formule suivante :

$$F_{90\%} = (\phi_{90\%} - \phi_{10\%})/((\phi_{90\%} + \phi_{10\%})/2)$$

dans laquelle $F_{90}$ est le facteur de distribution à 90 % ;

$\phi_{90\%}$ est la granulométrie maximale de la fraction de particules correspondant à 90 % desdites particules solides actives, et
$\phi_{10\%}$ est la granulométrie maximale de la fraction de particules correspondant à 10 % desdites particules solides actives.

**9.** Composition pharmaceutique orale contenant en tant qu'agent actif au moins un composé choisi dans le groupe constitué par :

un composé de formule (I)

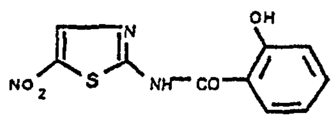
(I)

et un composé de formule (II)

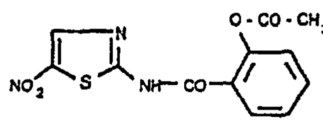
(II)

et une quantité améliorant la stabilité d'un acide pharmaceutiquement acceptable.

**10.** Composition selon la, revendication 9, dans laquelle l'acide pharmaceutiquement acceptable est choisi dans le groupe constitué par l'acide citrique, l'acide glutamique, l'acide succinique, l'acide éthanesulfonique, l'acide acétique, l'acide tartrique, l'acide ascorbique, l'acide méthanesulfonique, l'acide fumarique, l'acide adipique, l'acide malique et les mélanges de ceux-ci.

**11.** Composition selon la revendication 9, dans laquelle le rapport du poids de l'acide pharmaceutiquement acceptable au poids desdites particules solides actives est compris entre 0,01 et 0,5.

**12.** Composition selon la revendication 9, dans laquelle le rapport du poids de l'acide pharmaceutiquement acceptable au poids desdites particules solides actives est compris entre 0,03 et 0,2.

**13.** Utilisation d'au moins un composé choisi dans le groupe constitué par un composé de formule (I) :

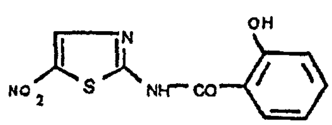
(I)

et un composé de formule (II) :

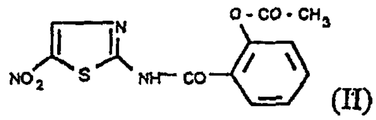

(II)

en tant qu'agent actif pour la fabrication d'une composition pharmaceutique destinée au traitement d'une infection chez un mammifère à système immunitaire compromis par un micro-organisme choisi dans le groupe constitué par Cryptosporidium pavum, Isopora belli, Enterocytzoon bieneusi, Encephlitozoon intestinalis, Mycobacterium tubreculosis, Myobacterium avium intracellulare, Pneumocystis carinii et Toxoplasma gondii, dans laquelle ledit agent actif est sous forme de particules actives ayant un granulométrie inférieure à 200 µm et une granulométrie moyenne supérieure à 10 µm.

**14.** Utilisation selon la revendication 13, dans laquelle ledit agent actif est sous forme de particules ayant une granulométrie moyenne comprise entre 20 et 50 µm.

**15.** Utilisation selon la revendication 13, dans laquelle ladite composition pharmaceutique contient au moins un acide pharmaceutiquement acceptable.

**16.** Utilisation selon la revendication 15, dans laquelle ledit acide pharmaceutiquement acceptable est choisi dans le groupe constitué par l'acide citrique, l'acide glutamique, l'acide succinique, l'acide éthanesulfonique, l'acide acétique, l'acide tartrique, l'acide ascorbique, l'acide méthanesulfonique, l'acide fumarique, l'acide adipique, l'acide malique et les mélanges de ceux-ci.

**17.** Utilisation selon la revendication 13, dans laquelle ledit agent actif est un composé de formule (I).

**18.** Utilisation selon la revendication 13, dans laquelle ledit agent actif est un composé de formule (II).

**19.** Utilisation selon la revendication 13, dans laquelle ledit mammifère est un être humain et dans laquelle ledit agent actif est administré en une quantité de 500 à 2000 mg par jour.

**20.** Utilisation d'au moins un composé choisi dans le groupe constitué par un composé de formule (I) :

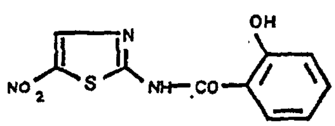

(I)

et un composé de formule (II) :

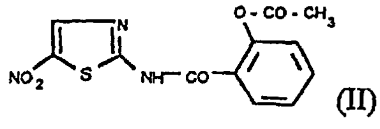

(II)

en tant qu'agent actif pour la fabrication d'une composition pharmaceutique destinée au traitement d'une infection parasitaire par un trématode, dans laquelle ledit agent actif est sous forme de particules actives ayant une granulométrie inférieure à 200 µm et une granulométrie moyenne supérieure à 10 µm.

**21.** Utilisation selon la revendication 20, dans laquelle le trématode est choisi dans le groupe constitué par Schistosoma, Fasciola, Fasciolopsis, Dicrocoelium, Heterephyes et Matagonimus.

**22.** Utilisation selon la revendication 21, dans laquelle le trématode est choisi dasn le groupe constitué par Schistosoma mansoni, Schistosoma haematobium, Schistosoma mekongi, Schistosoma japonicum, Schistosoma intercalatum, Fasciola hepatica, Fasciola gigantica, Fasciolopsis biski, Dicrocoelium dendriticum, Heterephyes heterophyes et Matagonimus yokogawa.

**23.** Utilisation selon la revendication 21, dans laquelle ledit agent actif est sous forme de particules ayant une granulométrie moyenne comprise entre 20 et 50 μm.

**24.** Utilisation selon la revendication 21, dans laquelle ladite composition pharmaceutique contient au moins un acide pharmaceutiquement acceptable.

**25.** Utilisation selon la revendication 24, dans laquelle ledit acide pharmaceutiquement acceptable est choisi dans le groupe constitué par l'acide citrique, l'acide glutamique, l'acide succinique, l'acide éthanesulfonique, l'acide acétique, l'acide tartrique, l'acide ascorbique, l'acide méthanesulfonique, l'acide fumarique, l'acide adipique, l'acide malique et les mélanges de ceux-ci.

**26.** Utilisation selon la revendication 21, dans laquelle le rapport du poids de l'acide pharmaceutiquement acceptable au poids desdites particules actives est compris entre 0,01 et 0,5.

**27.** Utilisation selon la revendication 21, dans laquelle ledit agent actif est un composé de formule (I).

**28.** Utilisation selon la revendication 21, dans laquelle ledit agent actif est un composé de formule (II).

**29.** Pâte pharmaceutique destinée à une administration locale, ladite pâte comprenant :

en tant qu'agent actif, des particules solides d'au moins un composé choisi dans le groupe constitué par :

un composé de formule (I)

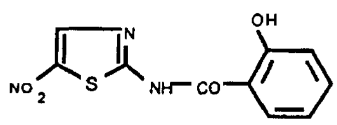

(I)

et un composé de formule (II)

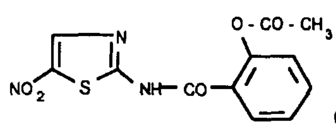

(II)

lesdites particules actives ayant une granulométrie inférieure à 200 μm et une granulométrie moyenne supérieure à 10 μm ;
au moins un épaississant ;
au moins un agent mouillant ; et
au moins un acide pharmaceutiquement acceptable, le pH de la pâte étant compris entre 2 et 6.

**30.** Pâte pharmaceutique selon la revendication 29, dans laquelle ladite pâte comprenden outre au moins un additif choisi dans le groupe constitué par l'alcool cétylique, les dérivés de glycéride, le propylèneglycol et les mélanges de ceux-ci.

**31.** Composition pharmaceutique destinée à une administration orale contenant un agent actif granulé en présence

d'un agent de granulation, dans laquelle :

ledit agent actif est sous forme de particules solides actives d'au moins un composé choisi dans le groupe constitué par :

un composé de formule (I)

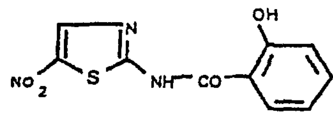

(I)

et un composé de formule (II)

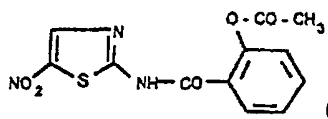

(II)

et dans laquelle lesdites particules actives ont une granulométrie inférieure à 200 μm et une granulométrie moyenne supérieure à 10 μm.

**32.** Composition selon la revendication 31, dans laquelle ledit agent de granulation est choisi dans le groupe constitué par la polyvinylpyrrolidone, l'eau, un alcool, le saccharose, l'hydroxylcellulose et les mélanges de ceux-ci.

**33.** Composition selon la revendication 31, dans laquelle lesdites particules solides actives granulées contiennent au moins un acide pharmaceutiquement acceptable.

**34.** Composition selon la revendication 31, dans laquelle ledit acide pharmaceutiquement acceptable est choisi dans le groupe constitué par l'acide citrique, l'acide glutamique, l'acide succinique, l'acide éthanesulfonique, l'acide acétique, l'acide tartrique, l'acide ascorbique, l'acide méthanesulfonique, l'acide fumarique, l'acide adipique, l'acide malique et les mélanges de ceux-ci.

**35.** Composition selon la revendication 33, dans laquelle le rapport du poids de l'acide pharmaceutiquement acceptable au poids dudit agent actif est compris entre 0,01 et 0,5.

**36.** Composition pharmaceutique destinée à une administration par voie orale contenant un agent actif, un agent mouillant et un dérivé d'amidon, dans laquelle :

ledit agent actif est sous forme de particules solides actives d'au moins un composé choisi dans le groupe constitué par :

un composé de formule (I)

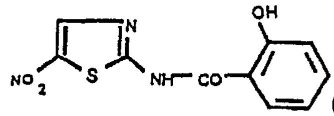

(I)

et un composé de formule (II)

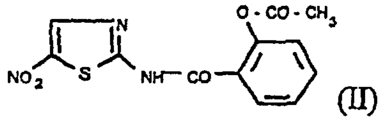

(II)

et dans laquelle lesdites particules actives ont une granulométrie inférieure à 200 µm et une granulométrie moyenne supérieure à 10 µm.

**37.** Composition pharmaceutique selon la revendication 36, comprenant en outre au moins un acide pharmaceutiquement acceptable.

**38.** Composition pharmaceutique selon la revendication 36, dans laquelle les particules actives sont granulées en présence d'un agent de granulation pour former un agent actif granulé contenant de 2 à 99,97 % en poids dudit composé actif et de 0,03 à 10 % en poids d'un agent de granulation.

**39.** Composition pharmaceutique selon la revendication 38, dans laquelle ledit agent de granulation est choisi dans le groupe constitué par la polyvinylpyrrolidone, l'eau, un alcool, le saccharose, l'hydroxylcellulose et les mélanges de ceux-ci.

**40.** Suspension liquide d'un agent actif destinée à une administration par voie orale, ladite suspension contenant :

en tant qu'agent actif, des particules solides d'au moins un composé choisi dans le groupe constitué par :

un composé de formule (I)

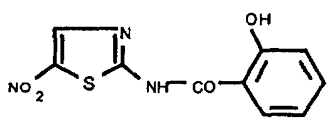

(I)

et un composé de formule (II)

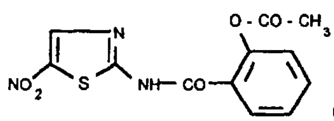

(II)

dans laquelle ledit agent actif est sous forme de particules actives ayant une granulométrie inférieure à 200 µm et une granulométrie moyenne supérieure à 10 µm ; et
au moins un acide pharmaceutiquement acceptable, le pH de la suspension étant compris entre 2 et 6.

**41.** Suspension selon la revendication 40, dans laquelle le pH de la suspension est compris entre 3 et 5.

**42.** Suspension selon la revendication 40, comprenant en outre un agent de granulation.

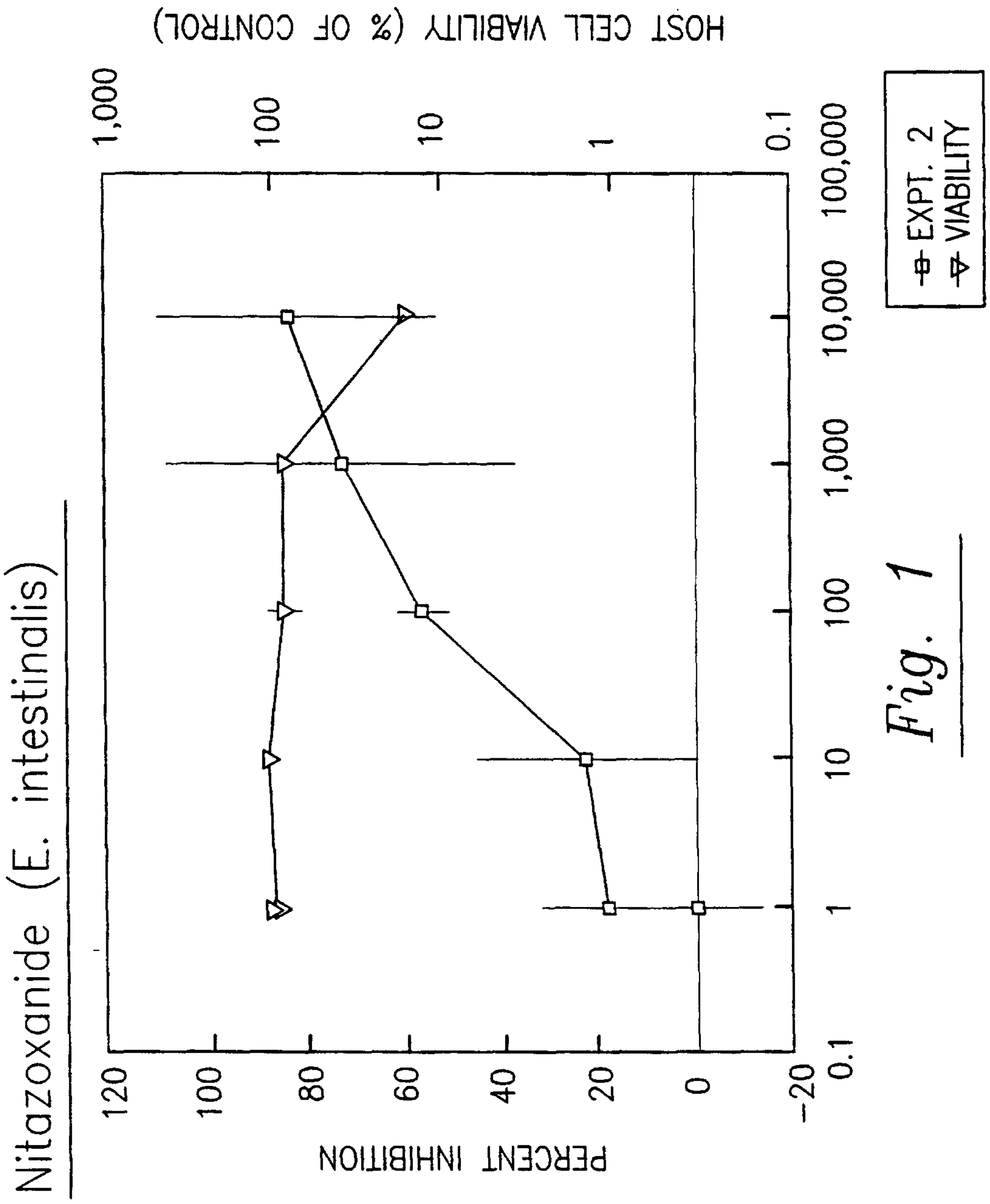
Nitazoxanide (E. intestinalis)
PERCENT INHIBITION
120
100
80
60
40
20
0
-20
0.1
1
10
100
1,000
10,000
100,000
HOST CELL VIABILITY (% OF CONTROL)
1,000
100
10
1
0.1
EXPT. 2
VIABILITY

*Fig. 1*

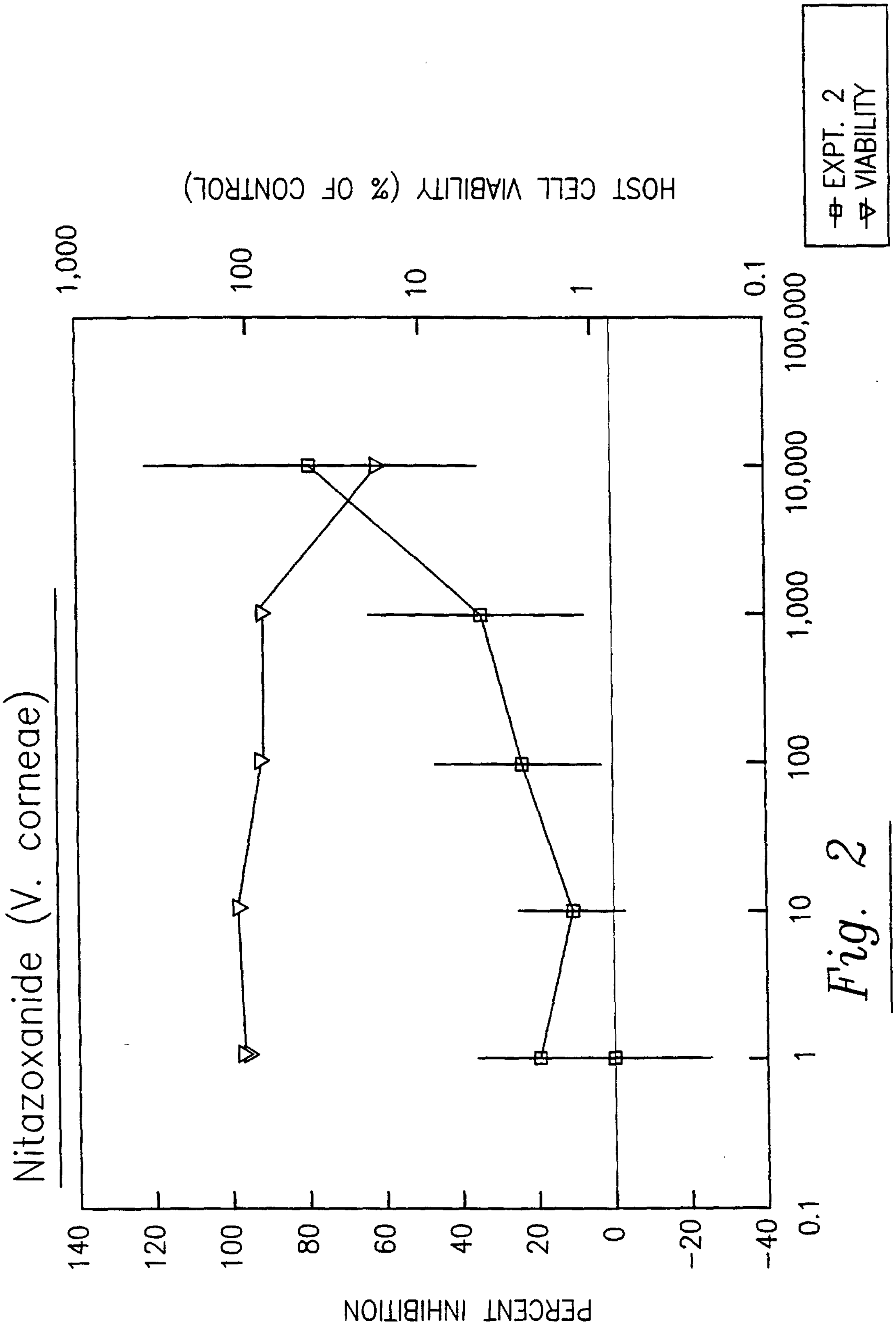
Nitazoxanide (V. corneae)
PERCENT INHIBITION
140
120
100
80
60
40
20
0
-20
-40
0.1
1
10
100
1,000
10,000
100,000
HOST CELL VIABILITY (% OF CONTROL)
1,000
100
10
1
0.1
EXPT. 2
VIABILITY

*Fig. 2*

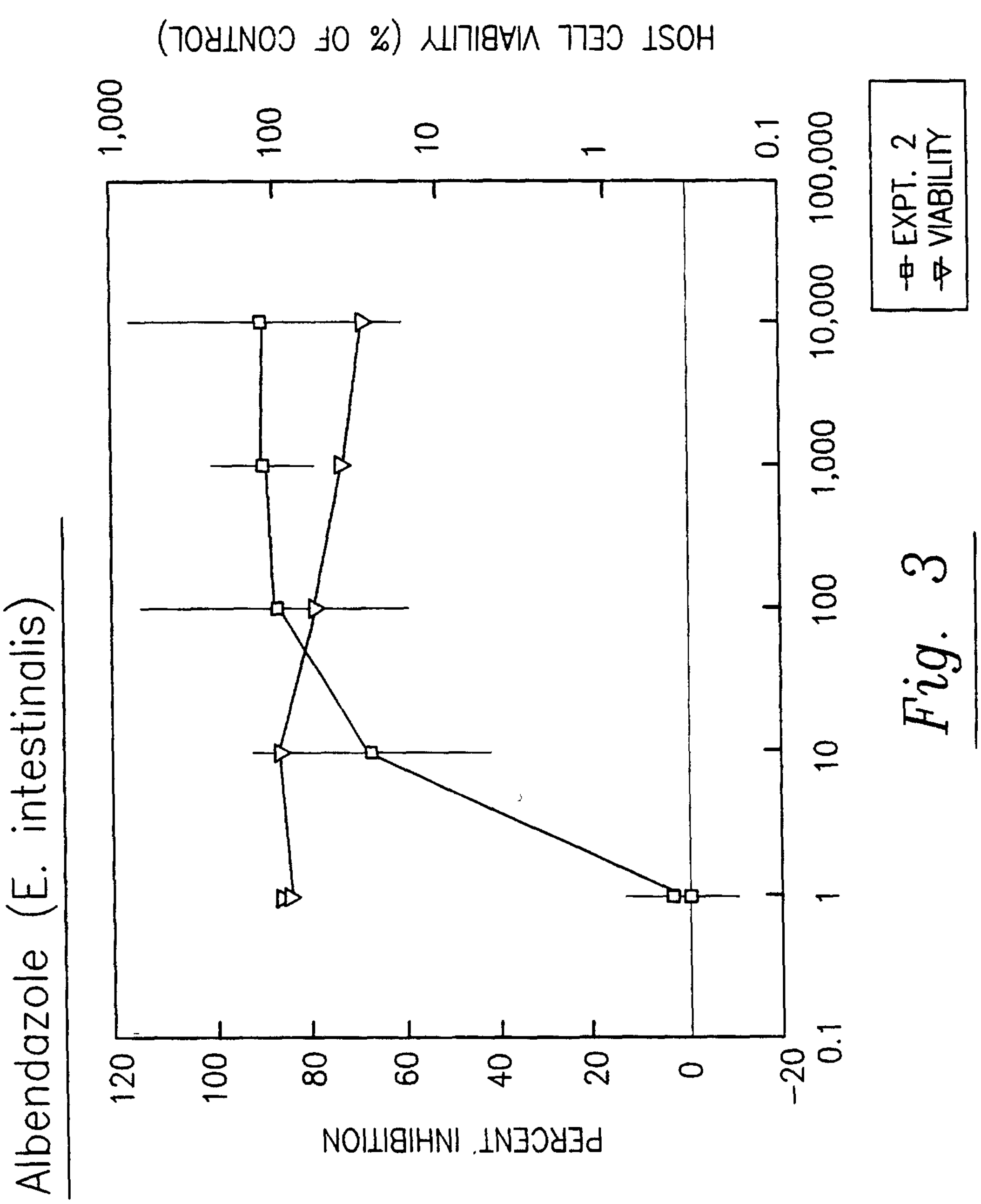
Albendazole (E. intestinalis)
PERCENT INHIBITION
120
100
80
60
40
20
0
-20
0.1
1
10
100
1,000
10,000
100,000
HOST CELL VIABILITY (% OF CONTROL)
1,000
100
10
1
0.1
EXPT. 2
VIABILITY

*Fig. 3*

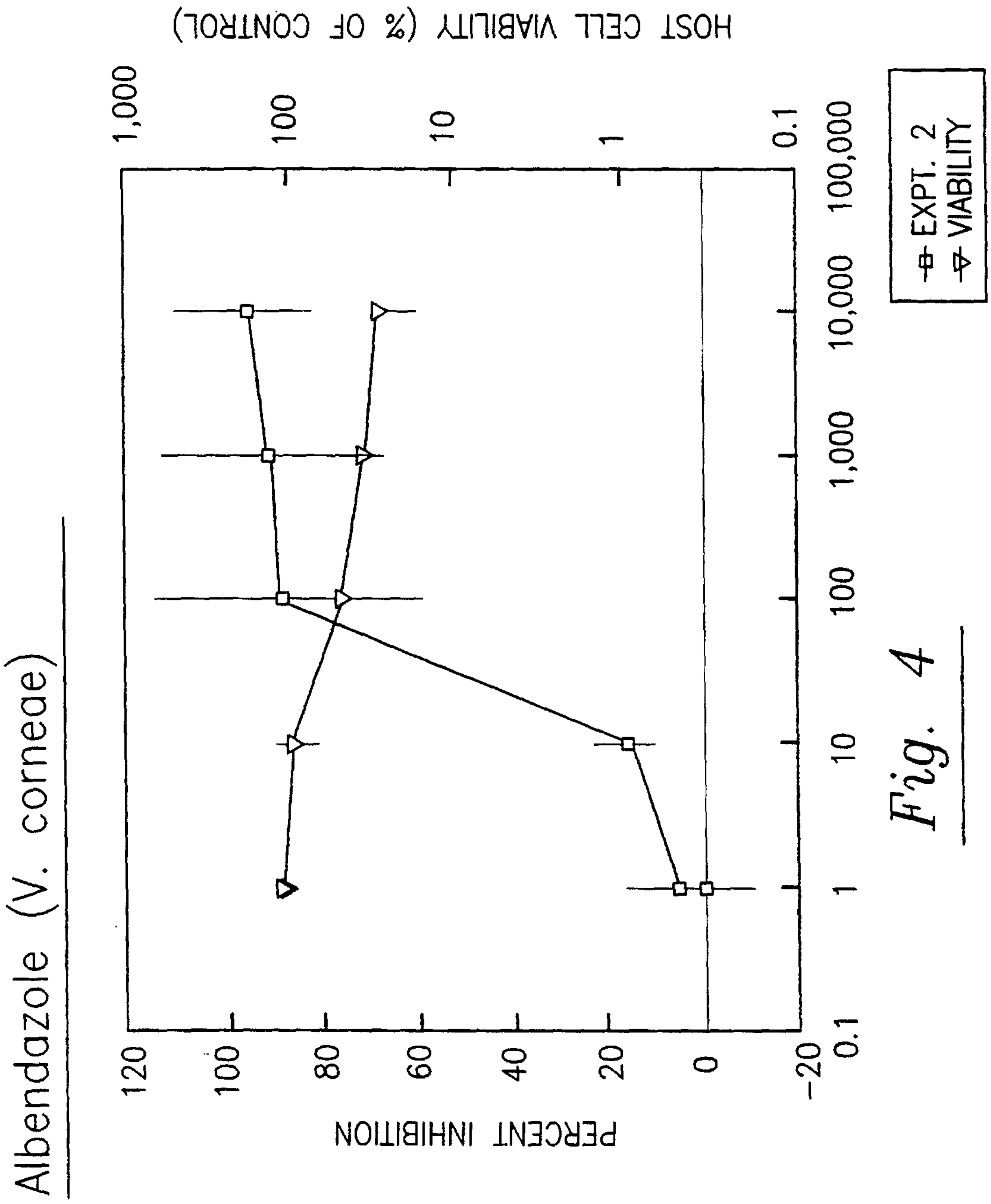
Albendazole (V. corneae)
PERCENT INHIBITION
120
100
80
60
40
20
0
−20
0.1
1
10
100
1,000
10,000
100,000
HOST CELL VIABILITY (% OF CONTROL)
1,000
100
10
1
0.1
EXPT. 2
VIABILITY

*Fig. 4*

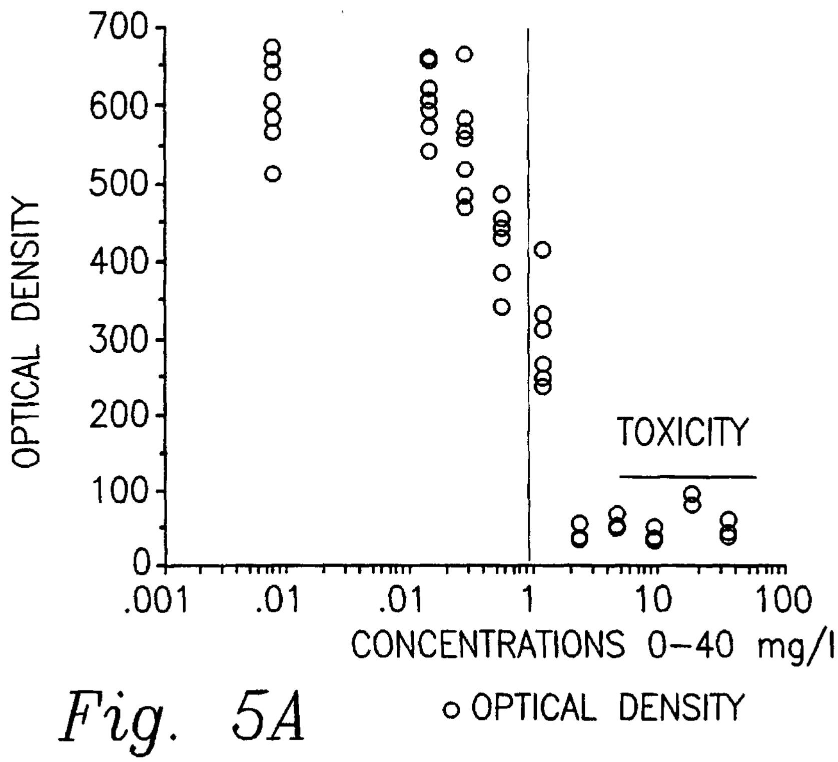
700
600
500
400
300
200
100
0
OPTICAL DENSITY
TOXICITY
.001
.01
.01
1
10
100
CONCENTRATIONS 0–40 mg/l
○ OPTICAL DENSITY

*Fig. 5A*

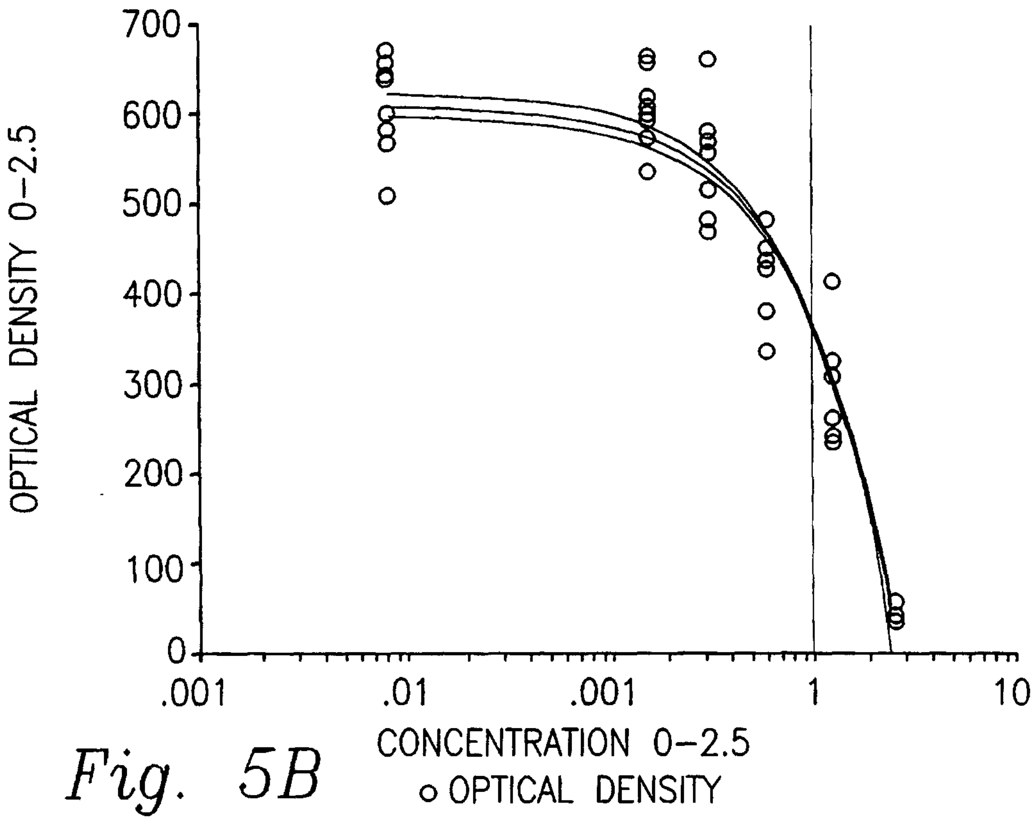
700
600
500
400
300
200
100
0
OPTICAL DENSITY 0–2.5
.001
.01
.001
1
10
CONCENTRATION 0–2.5
○ OPTICAL DENSITY

*Fig. 5B*

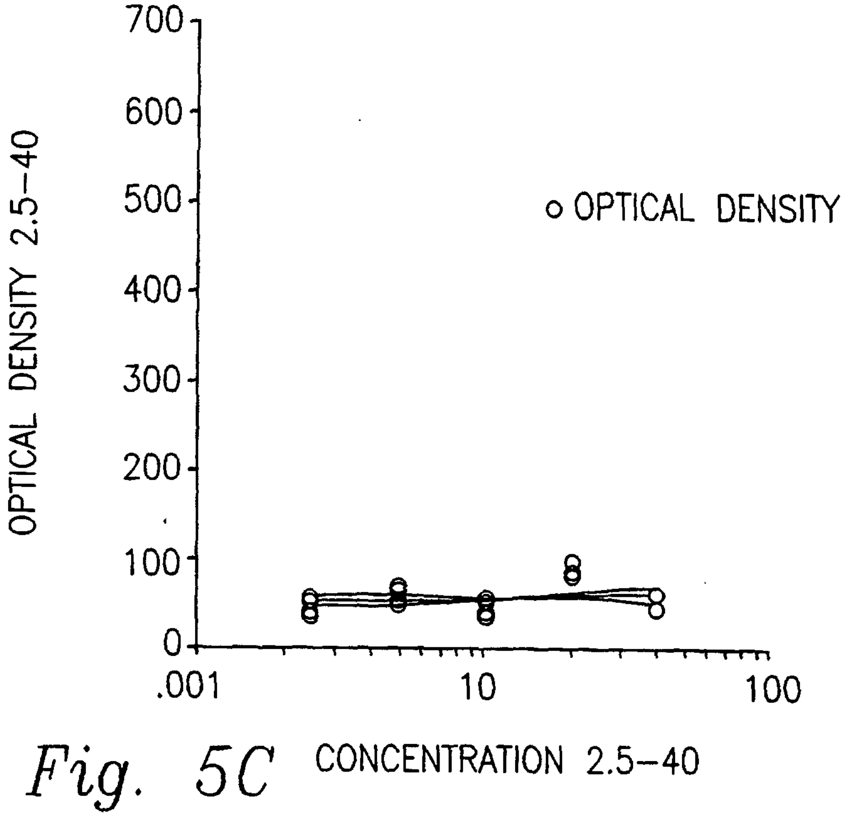
OPTICAL DENSITY 2.5−40
700
600
500
400
300
200
100
0
.001
10
100
○ OPTICAL DENSITY
CONCENTRATION 2.5−40

*Fig. 5C*

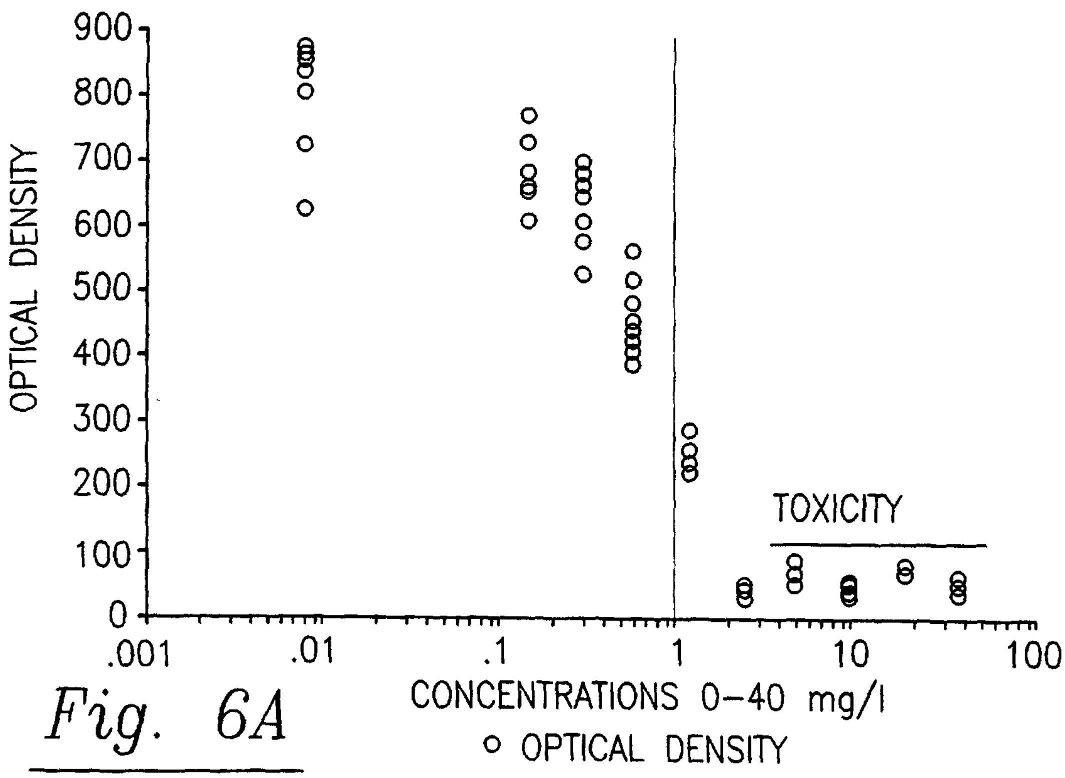
OPTICAL DENSITY
900
800
700
600
500
400
300
200
100
0
.001
.01
.1
1
10
100
TOXICITY
CONCENTRATIONS 0−40 mg/l
○ OPTICAL DENSITY

*Fig. 6A*

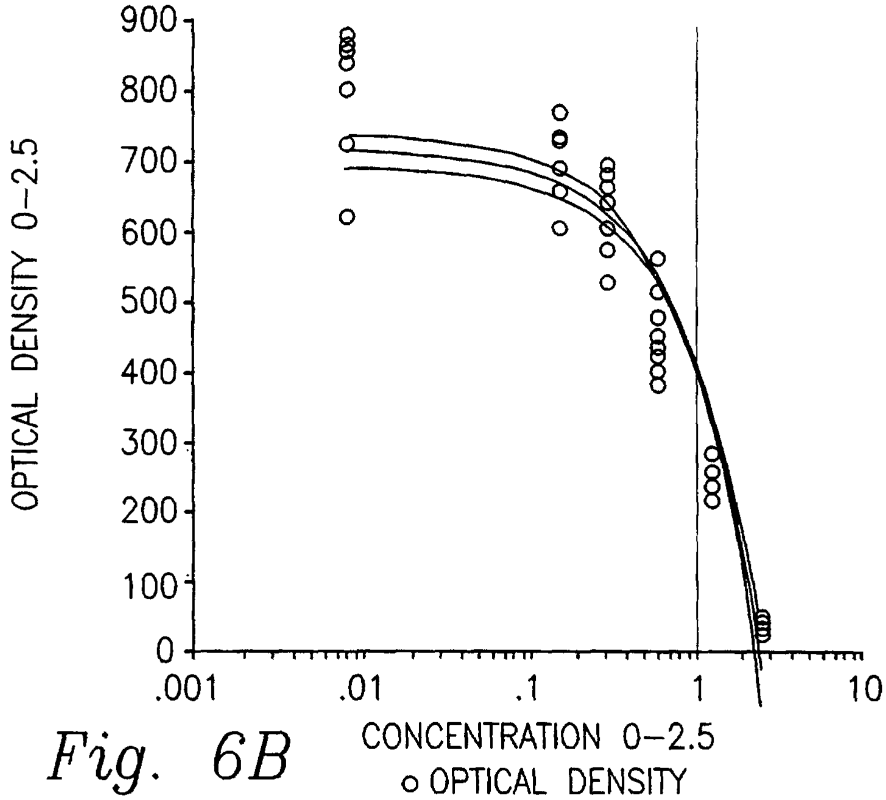
900
800
700
600
500
400
300
200
100
0
OPTICAL DENSITY 0–2.5
.001
.01
.1
1
10
CONCENTRATION 0–2.5
o OPTICAL DENSITY

Fig. 6B

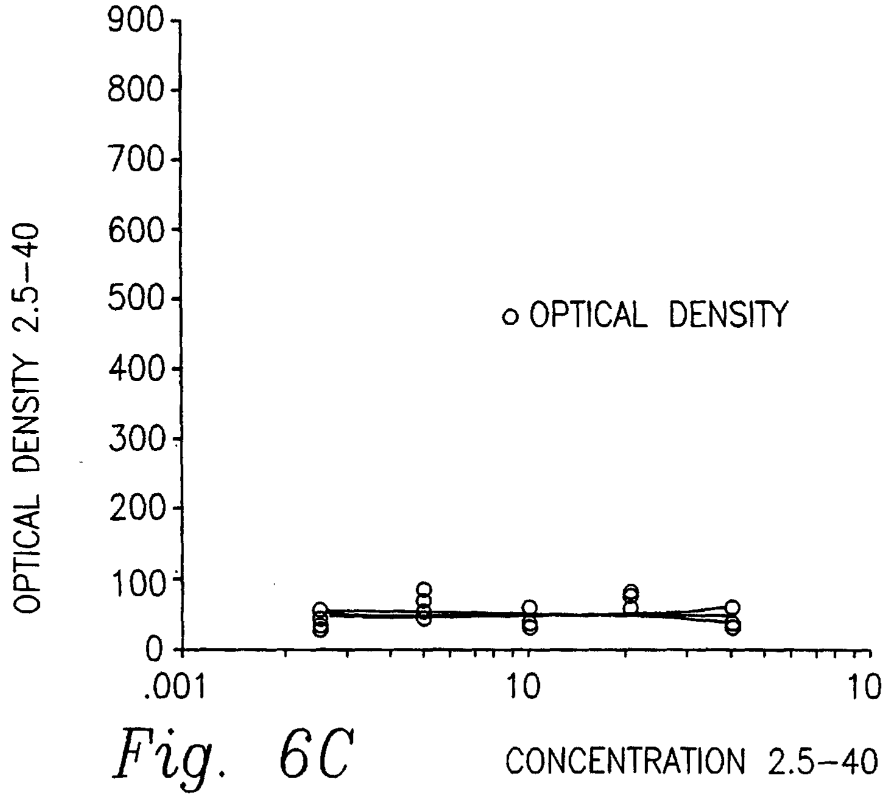
900
800
700
600
500
400
300
200
100
0
OPTICAL DENSITY 2.5–40
o OPTICAL DENSITY
.001
10
10
CONCENTRATION 2.5–40

Fig. 6C

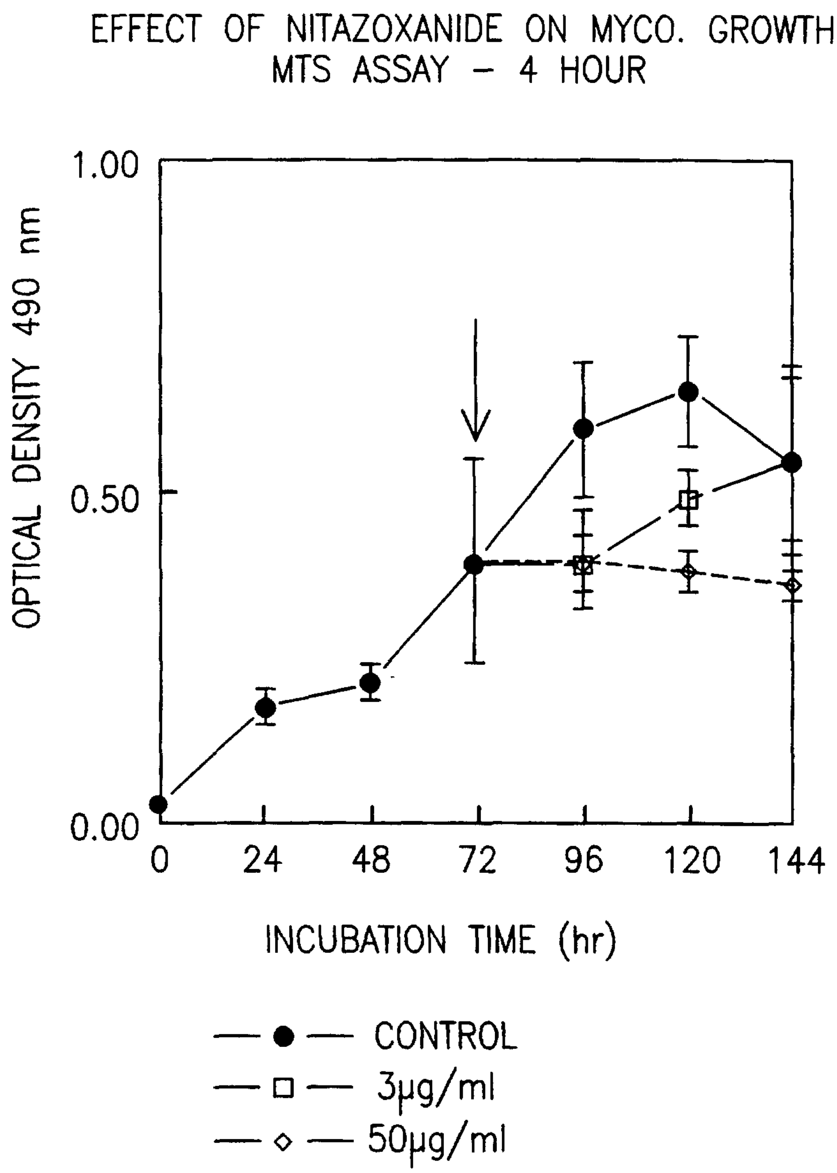
EFFECT OF NITAZOXANIDE ON MYCO. GROWTH
MTS ASSAY - 4 HOUR
OPTICAL DENSITY 490 nm
1.00
0.50
0.00
0
24
48
72
96
120
144
INCUBATION TIME (hr)
CONTROL
3µg/ml
50µg/ml

*Fig. 7*

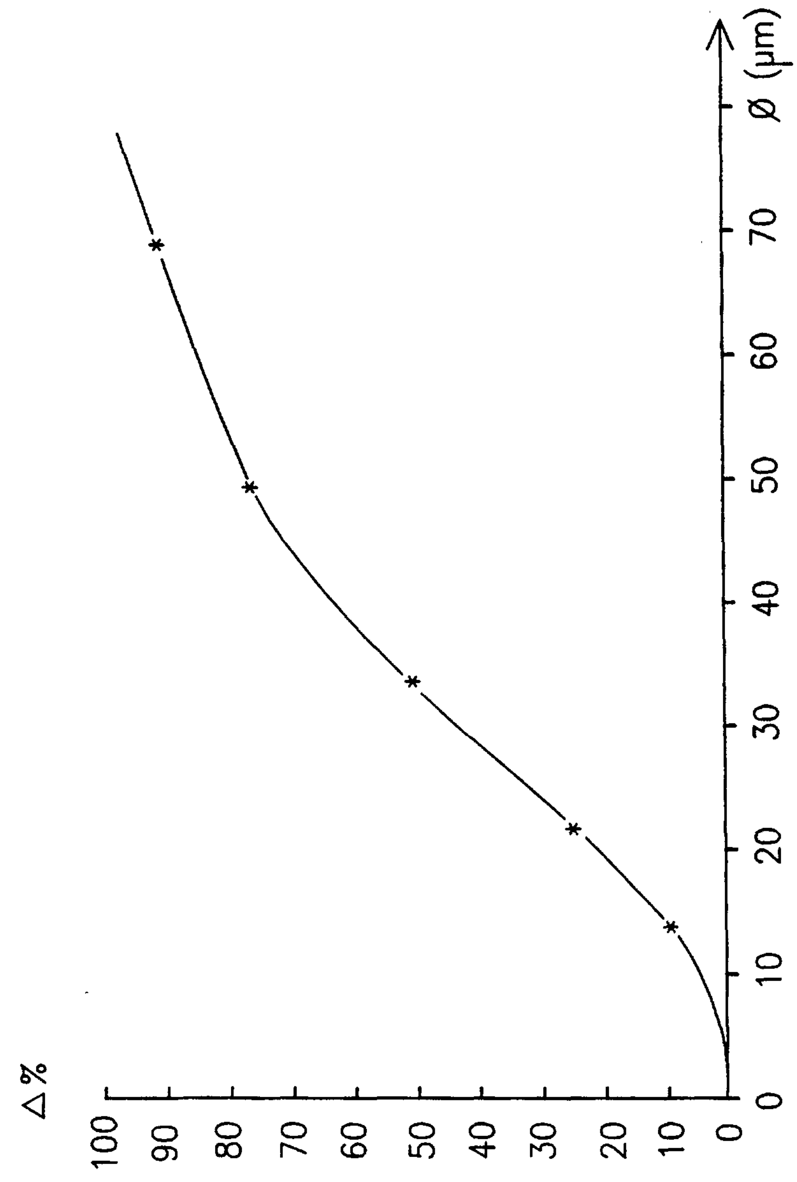


*Fig. 8*